# EUROPEAN PATENT APPLICATION

(11) **EP 4 397 654 A1**
(43) Date of publication of application: **10.07.2024**
(21) Application number: 22863626.2
(22) Date of filing: 02.09.2022
(51) Int. Cl.: C07D 231/20, A61K 31/415, A61P 35/00, A61P 29/00, A61P 19/02, A61P 15/00, A61P 3/00, A61P 13/10

(54) **EP4 ANTAGONIST COMPOUND AS WELL AS SALT, POLYMORPH, AND USE THEREOF**

(30) Priority: 03.09.2021 CN 202111034159
(71) Applicant: Wuhan Humanwell Innovative Drug Research and Development Center Limited Company, Wuhan, Hubei 430075 (CN)
(72) Inventor: ZHANG, Xuejun, Wuhan, Hubei 430075 (CN); YUE, Yang, Wuhan, Hubei 430075 (CN); ZANG, Yang, Wuhan, Hubei 430075 (CN); LEI, Sijun, Wuhan, Hubei 430075 (CN); XIA, Qingfeng, Wuhan, Hubei 430075 (CN); LI, Yuan, Wuhan, Hubei 430075 (CN); LIU, Zhe, Wuhan, Hubei 430075 (CN); YANG, Jun, Wuhan, Hubei 430075 (CN); LI, Li'e, Wuhan, Hubei 430075 (CN)
(74) Representative: Gille Hrabal Partnerschaftsgesellschaft mbB Patentanwälte
(86) International application number: PCT/CN2022/116768
(87) International publication number: WO 2023/030492

(57) **Abstract**

An EP4 antagonist represented by formula I, which specifically comprises a crystal form thereof, a pharmaceutically acceptable salt or crystal form thereof, a preparation method therefor, a composition containing same, and a medical use of a related compound. The structure of formula I is shown below:

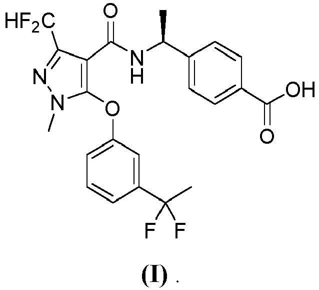

## Description

The present application claims priority to the prior application with the patent application No. 202111034159.1 and entitled "EP4 ANTAGONIST COMPOUND AS WELL AS SALT, POLYMORPH, AND USE THEREOF" filed to the China National Intellectual Property Administration on Sep. 3, 2021, which is incorporated herein by reference in its entirety.

### TECHNICAL FIELD

The present disclosure belongs to the field of pharmaceuticals, and relates to an EP4 antagonist compound as well as a salt and a polymorph thereof, a preparation method therefor, and use thereof.

### BACKGROUND

Prostaglandin E₂ (PGE₂) is an endogenous bioactive lipid. PGE₂ elicits a wide range of upstream and downstream dependent biological responses by activating prostaglandin receptors (Legler, D. F. et al., hit. J Biochem. Cell Biol. 2010, 42, p. 198-201), and is involved in the regulation of numerous physiological and pathological processes including inflammation, pain, renal function, cardiovascular system, pulmonary function, cancers, and the like. It has been reported that PGE₂ is highly expressed in cancerous tissues of various cancers, and it has been confirmed that **PGE**₂ is associated with the occurrence, growth, and progression of cancer and disease conditions in patients. It is generally believed that PGE₂ is associated with the activation of cell proliferation and cell death (apoptosis) and plays an important role in the processes of cancer cell proliferation, disease progression, and cancer metastasis.

Among the receptors of PGE₂, there are 4 subtypes, EP1, EP2, EP3, and EP4, which are widely distributed in various tissues. Among these subtypes, PGE₂ interferes with inflammatory responses (including immune inflammatory responses), smooth muscle relaxation, pain, lymphocyte differentiation, hypertrophy and proliferation of vascular mesangial cells, secretion of gastrointestinal mucus, and the like, via the EP4 receptor. Thus, it can be considered that EP4 receptor antagonists are promising as anti-inflammatory and/or analgesic agents for the treatment of diseases associated with the PGE₂-EP4 pathway, such as inflammatory diseases, diseases accompanied by various pains, and the like.

EP4 is the major receptor involved in arthritic pain in rodent models of rheumatoid arthritis and osteoarthritis (see e.g., J. Pharmacol. Exp. Ther., 325, 425 (2008)), which upon activation leads to the accumulation of an intracellular signaling molecule cAMP. Studies have detected the expression of the EP4 receptor on peripheral nerve endings of nociceptors, macrophages, and neutrophils, and these cell types have been proven to be extremely important for endometriosis. Studies have reported that oral EP4 antagonists may reduce proteinuria in mice with type 2 diabetes, thereby inhibiting the progression of diabetic nephropathy. Additional studies have reported that the activation of EP4 and increased production of PGE₂ in the bladder mucosa may be important causes of overactive bladder caused by prostatitis, and intravesical injection of EP4 antagonists may be effective in improving overactive bladder after prostatitis. Therefore, selective EP4 antagonists may be useful in the treatment of arthritis, including arthritis pain as well as endometriosis, diabetic nephropathy, and overactive bladder. Existing therapeutic drugs for arthritis are mainly conventional non-steroidal anti-inflammatory drugs (NSAIDs) or selective COX-2 inhibitors, which may produce cardiovascular and/or gastrointestinal side effects. However, the selective EP4 antagonists are less likely to produce cardiovascular side effects.

PGE₂ continuously activates EP receptors (abundantly produced by tumor cells) in the tumor microenvironment (Ochs et al., J Neurochem., 2016, 136, p. 1142-1154; Zelenay, S. et al., Cell, 2015, 162, p. 1257-1270), which promotes the accumulation of a variety of immunosuppressive cells and enhances the activity thereof. The immunosuppressive cells include type 2 tumor-associated macrophages (TAMs), Treg cells, and myeloid-derived suppressor cells (MDSCs). One of the main features of the immunosuppressive tumor microenvironment is the presence of a large number of MDSCs and TAMs, which in turn are closely associated with low overall survival rate in patients with gastric cancer, ovarian cancer, breast cancer, bladder cancer, hepatocellular carcinoma (HCC), head and neck cancer, and other types of cancer. In addition, it has been reported that PGE₂ induces immune tolerance by inhibiting the accumulation of antigen-presenting dendritic cells (DCs) in tumors and inhibiting the activation of tumor-infiltrating DCs (Wang et al., Trends in Molecular Medicine, 2016, 22, p. 1-3). All of these PGE₂-mediated effects will collectively help tumor cells evade immune surveillance. PGE₂ plays an important role in promoting the development and progression of tumors. Increased expression levels of PGE₂ and its related receptors EP2 and EP4 have been found in various malignant tumors, including colon cancer, lung cancer, breast cancer, head and neck cancer, and the like, and they are often closely associated with poor prognosis (Bhooshan, N. et al., Lung Cancer 101, 88-91). Therefore, selective blockade of EP2 and EP4 signaling pathways may inhibit the development and progression of tumors by altering the tumor microenvironment and regulating tumor immune cells.

Existing preclinical research data show that EP2- and EP4-specific antagonists may prevent or inhibit the growth of tumors to different degrees in animal models of colon cancer, esophageal cancer, lung cancer, breast cancer, and the like. In the PGE₂ receptor drugs entering the clinic, Grapiprant, an EP4 antagonist developed by Pfizer, has been approved by the FDA for the treatment of arthritis in dogs, and meanwhile, it entered anti-tumor phase II clinical trials in 2015 for the treatment of various types of solid tumors such as prostate cancer, non-small cell lung cancer, and breast cancer (De Vito, V. et al., J Pharm Biomed Anal, 118, 251-258). A related clinical phase I study on E7046, an EP4 antagonist developed by Eisai, was also started in 2015, and a phase Ib clinical trial for treating rectal cancer in combination with radiotherapy or chemoradiotherapy was developed in 2017. ONO-4578, developed by Ono Pharmaceutical, entered clinical phase I studies on advanced or metastatic solid tumors in 2017, and entered phase VII clinical trials for the treatment of advanced solid tumors alone or in combination with nivolumab in 2018.

Chinese Patent Application CN202110240347.3 discloses the following structure of formula I:

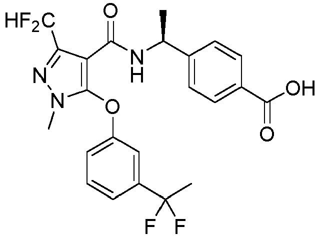

The compound of formula I is capable of effectively antagonizing the activity of an EP4 receptor, and has wide application prospects for the manufacturing of a medicament for treating diseases associated with EP4, so that further study on the compound of formula I as well as salt forms and crystal forms thereof is of great significance in developing effective therapeutic medicaments.

### SUMMARY

In order to solve the problems in the prior art, in one aspect, the present disclosure provides a crystal form of a compound of formula I, wherein the structure of formula I is shown below:

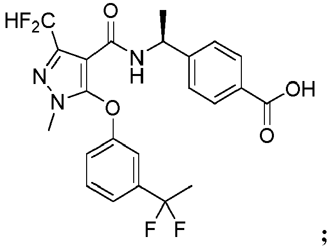

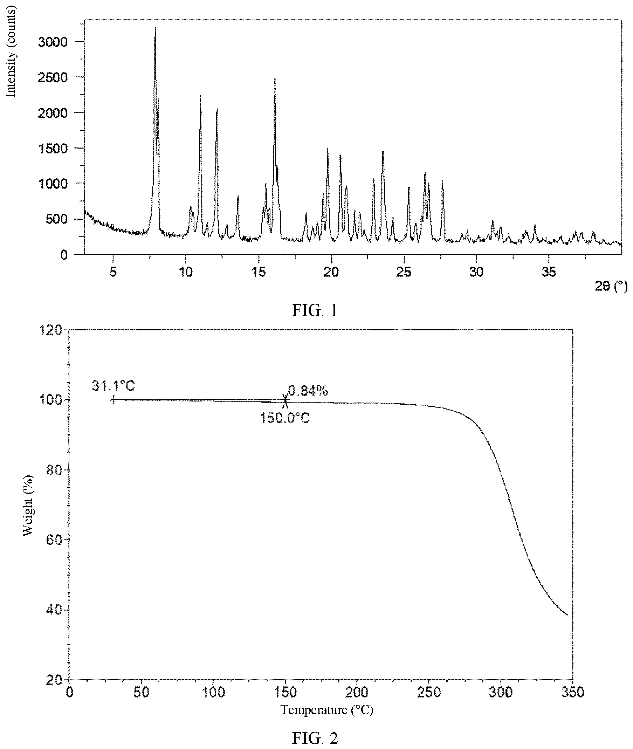

In some embodiments, the present disclosure provides free acid crystal form A of the compound of formula I, and the free acid crystal form A has an X-ray powder diffraction pattern comprising diffraction peaks at 2θ±0.2° diffraction angles of 7.88, 11.00, 12.13, 16.10, 19.75, 20.65, 21.04, 22.92, 23.53, and 26.69; or the free acid crystal form A has an X-ray powder diffraction pattern comprising diffraction peaks at 2θ±0.2° diffraction angles of 7.88, 8.08, 11.00, 12.13, 16.10, 19.75, 20.65, and 23.53; further, the free acid crystal form A has an X-ray powder diffraction pattern comprising diffraction peaks at 2θ±0.2° diffraction angles of 7.88, 8.08, 11.00, 12.13, 13.59, 15.50, 16.10, 19.44, 19.75, 20.65, 21.04, 22.92, 23.53, 25.32, 26.44, 26.69, and 27.67; furthermore, the free acid crystal form A has an X-ray powder diffraction pattern comprising diffraction peaks at 2θ±0.2° diffraction angles of 7.88, 8.08, 10.31, 10.49, 11.00, 11.46, 12.13, 12.82, 13.59, 15.29, 15.50, 15.72, 16.10, 18.27, 18.70, 19.04, 19.44, 19.75, 20.65, 21.04, 21.62, 21.97, 22.27, 22.92, 23.53, 24.22, 25.32, 25.81, 26.21, 26.44, 26.69, 27.67, 28.99, 29.35, 30.17, 31.12, 31.65, 32.18, 33.44, 33.97, 35.78, 36.84, 37.18, and 37.99; still further, the free acid crystal form A has an XRPD pattern substantially shown in FIG. 1.

In some embodiments, the free acid crystal form A has one, two, or three of the following characteristics:
(1) a TGA curve of the free acid crystal form A showing a weight loss of about 0-2%, preferably about 0-1% (e.g., 0.84%) at 150.0±3 °C;
(2) a DSC curve of the free acid crystal form A having a starting point of an endothermic peak at 134.0±3 °C; and
(3) the DSC curve of the free acid crystal form A having an endothermic peak at 136.8±3 °C.

In some embodiments, the free acid crystal form A has one, two, or three of the following characteristics:
(1) the TGA curve of the free acid crystal form A showing a weight loss of about 0.84% at 150.0±3 °C;
(2) the DSC curve of the free acid crystal form A having a starting point of an endothermic peak at 134.0±3 °C; and
(3) the DSC curve of the free acid crystal form A having an endothermic peak at 136.8±3 °C.

In some embodiments, the TGA profile of the free acid crystal form A is shown in FIG. 2; the DSC profile of the free acid crystal form A is shown in FIG. 3; a ¹H NMR spectrum of the free acid crystal form A is shown in FIG. 4.

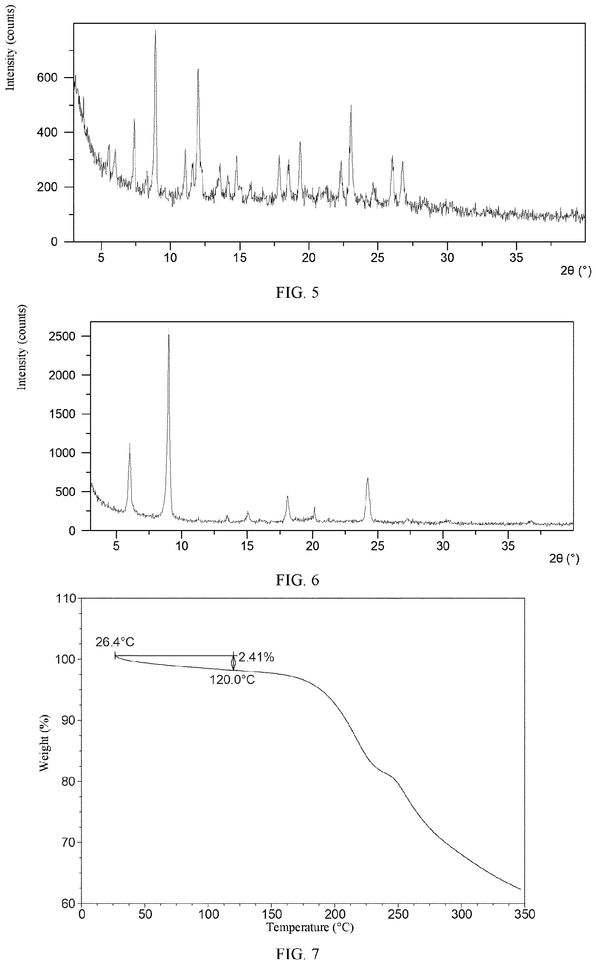

In some embodiments, the present disclosure provides free acid crystal form B of the compound of formula I, and the free acid crystal form B has an X-ray powder diffraction pattern comprising diffraction peaks at 2θ±0.2° diffraction angles of 7.38, 8.91, 11.07, 17.85, 18.52, 19.38, 23.05, 26.01, and 26.76; further, the free acid crystal form B has an X-ray powder diffraction pattern comprising diffraction peaks at 2θ±0.2° diffraction angles of 7.38, 8.91, 11.07, 12.01, 17.85, 18.52, 19.38, 23.05, 26.01, and 26.76; further, the free acid crystal form B has an X-ray powder diffraction pattern comprising diffraction peaks at 2θ±0.2° diffraction angles of 5.56, 6.00, 7.38, 8.91, 11.07, 11.58, 12.01, 13.58, 14.16, 14.78, 17.85, 18.52, 19.38, 22.33, 23.05, 24.64, 26.01, and 26.76; furthermore, the free acid crystal form B has an X-ray powder diffraction pattern comprising diffraction peaks at 2θ±0.2° diffraction angles of 5.56, 6.00, 7.38, 8.27, 8.91, 11.07, 11.58, 12.01, 13.58, 14.16, 14.78, 15.73, 17.85, 18.52, 19.38, 22.33, 23.05, 24.64, 26.01, and 26.76; still further, the free acid crystal form B has an XRPD pattern substantially shown in FIG. 5.

In another aspect, the present disclosure provides a pharmaceutically acceptable salt of the compound of formula I, which may be selected from an alkali metal salt (e.g., a sodium salt or potassium salt), an alkaline earth metal salt (e.g., a calcium salt or magnesium salt), or an ammonium salt of the compound of formula I, or may be selected from a salt formed with an organic base that provides a physiologically acceptable cation, such as a salt formed with the following bases: sodium hydroxide, potassium hydroxide, calcium hydroxide, magnesium hydroxide, zinc hydroxide, betaine, monoethanolamine, caffeine, urea, nicotinamide, isonicotine, dimethylglucamine, ethylglucamine, glucosamine, meglumine, lysine, arginine, choline, aqueous ammonia, dicyclohexylamine, 1,6-hexanediamine, ethanolamine, diethanolamine, sarcosine, serinol, tris(hydroxymethyl)aminomethane, aminopropanediol, tromethamine, diethylamine, and imidazole. According to a preferred embodiment of the present disclosure, the pharmaceutically acceptable salt of the compound of formula I is a tromethamine salt, a diethylamine salt, and a lysine salt of the compound of formula I.

According to an embodiment of the present disclosure, it will be understood by those skilled in the art that when the compound of formula I forms a salt with a base, the compound of formula I and the base may be in a molar ratio of 5:1 to 1:5, such as 3:1, 2:1, 1:1, 1:1.5, 1:2, 1:2.5, or 1:3. Preferably, the compound of formula I and the base are in a molar ratio of 1:1.

In another aspect, the present disclosure provides a crystal form of the pharmaceutically acceptable salt of the compound of formula I.

In some embodiments, the present disclosure provides crystal form A of the tromethamine salt of the compound of formula I, and the crystal form A of the tromethamine salt has an X-ray powder diffraction pattern comprising diffraction peaks at 2θ±0.2° diffraction angles of 6.03, 9.01, 13.50, 15.06, 18.09, and 24.27; further, the crystal form A of the tromethamine salt has an X-ray powder diffraction pattern comprising diffraction peaks at 2θ±0.2° diffraction angles of 6.03, 9.01, 13.50, 15.06, 16.03, 18.09, 24.27, 27.28, 30.39, and 36.72; furthermore, the crystal form A of the tromethamine salt has an XRPD pattern substantially shown in FIG. 6.

In some embodiments, the crystal form A of the tromethamine salt has one, two, or three of the following characteristics:
(1) a TGA curve of the crystal form A of the tromethamine salt showing a weight loss of about 1.0%-3.5%, preferably about 2.0%-3.0% (e.g., 2.41%) at 120.0±3 °C;
(2) a DSC curve of the crystal form A of the tromethamine salt having a starting point of an endothermic peak at 124.5±3 °C; and
(3) the DSC curve of the free acid crystal form A having an endothermic peak at 139.2±3 °C.

In some embodiments, the crystal form A of the tromethamine salt has one, two, or three of the following characteristics:
(1) the TGA curve of the crystal form A of the tromethamine salt showing a weight loss of about 2.41% at 120.0±3 °C;
(2) the DSC curve of the crystal form A of the tromethamine salt having a starting point of an endothermic peak at 124.5±3 °C; and
(3) the DSC curve of the free acid crystal form A having an endothermic peak at 139.2±3 °C.

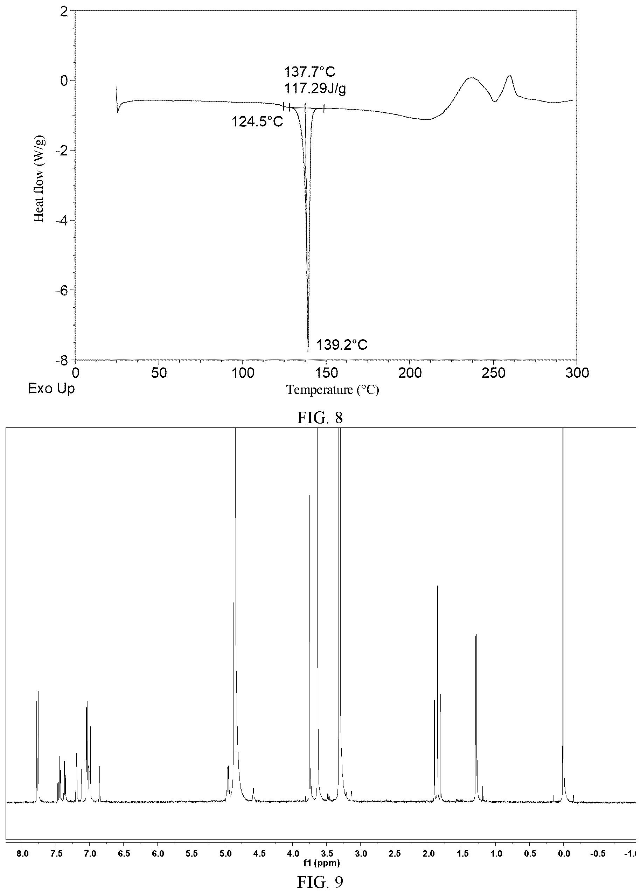

In some embodiments, the TGA profile of the crystal form A of the tromethamine salt is shown in FIG. 7; the DSC profile of the crystal form A of the tromethamine salt is shown in FIG. 8; a ¹H NMR spectrum of the crystal form A of the tromethamine salt is shown in FIG. 9.

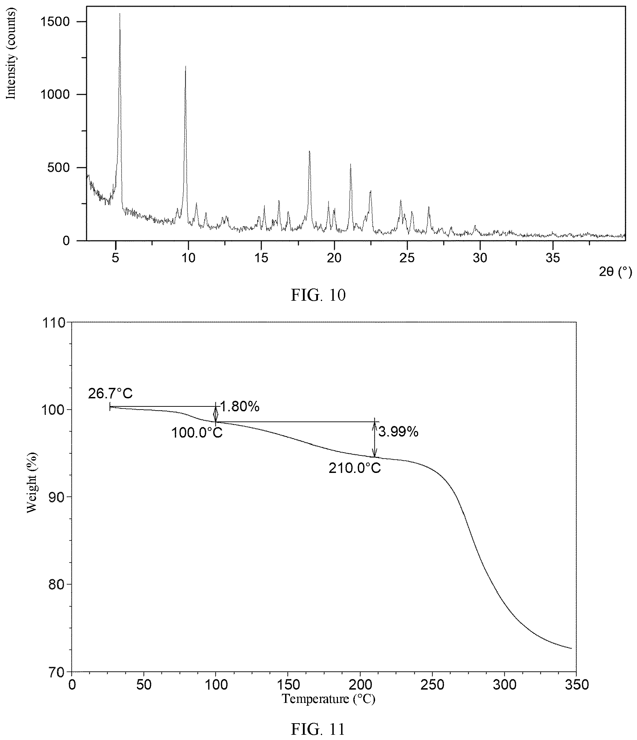

In some embodiments, the present disclosure provides crystal form A of the diethylamine salt of the compound of formula I, and the crystal form A of the diethylamine salt has an X-ray powder diffraction pattern comprising diffraction peaks at 2θ±0.2° diffraction angles of 5.29, 9.79, 10.53, 18.30, 19.61, 19.99, 21.10, 25.33, and 26.45; further, the crystal form A of the diethylamine salt has an X-ray powder diffraction pattern comprising diffraction peaks at 2θ±0.2° diffraction angles of 5.29, 9.79, 10.53, 11.20, 12.52, 14.85, 15.20, 16.22, 16.86, 18.30, 19.61, 19.99, 21.10, 22.12, 22.47, 24.57, 24.85, 25.33, 26.45, 27.39, 28.01, 29.61, 32.13, 35.00, and 37.44; furthermore, the crystal form A of the diethylamine salt has an XRPD pattern substantially shown in FIG. 10.

In some embodiments, the crystal form A of the diethylamine salt has one or two of the following characteristics:
(1) a TGA curve of the crystal form A of the diethylamine salt showing a weight loss of about 0.50%-3.00%, preferably about 1.00%-2.50% (e.g., 1.80%) at 100.0±3 °C; and showing a weight loss of about 2.00%-5.00%, preferably about 3.00%-4.50% (e.g., 3.99%) at 210.0±3 °C; and
(2) a DSC curve of the crystal form A of the diethylamine salt having two endothermic peaks at 104.3±10 °C and 121.5±10 °C; particularly, the DSC curve of the crystal form A of the diethylamine salt having two endothermic peaks at 104.3±5 °C and 121.5±5 °C.

In some embodiments, the crystal form A of the diethylamine salt has one or two of the following characteristics:
(1) the TGA curve of the crystal form A of the diethylamine salt showing a weight loss of about 1.80% at 100.0±3 °C and a weight loss of about 3.99% at 210.0±3 °C;
(2) the DSC curve of the crystal form A of the diethylamine salt having two endothermic peaks at 104.3±10 °C and 121.5±10 °C; particularly, the DSC curve of the crystal form A of the diethylamine salt having two endothermic peaks at 104.3±5 °C and 121.5±5 °C.

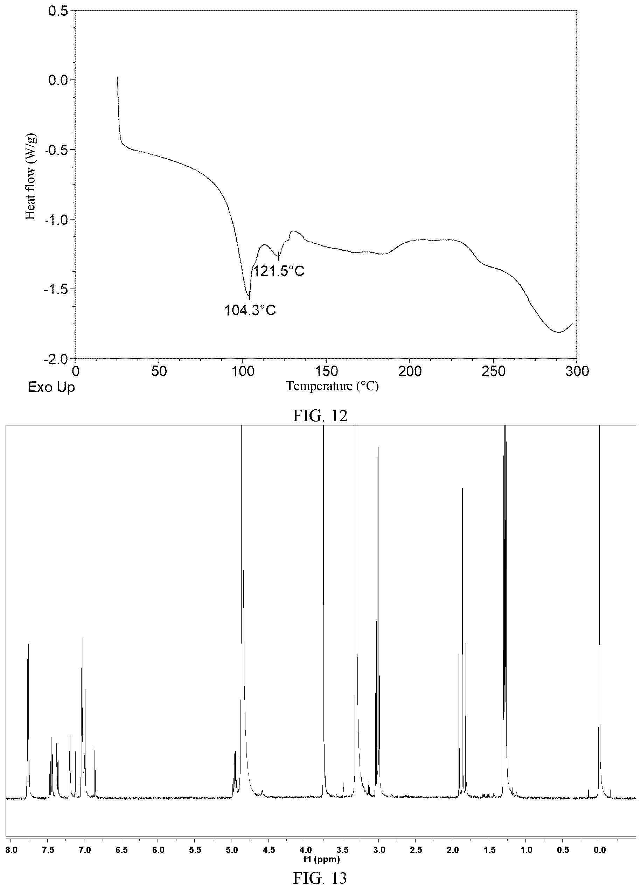

In some embodiments, the TGA profile of the crystal form A of the diethylamine salt is shown in FIG. 11; the DSC profile of the crystal form A of the diethylamine salt is shown in FIG. 12; a ¹H NMR spectrum of the crystal form A of the diethylamine salt is shown in FIG. 13.

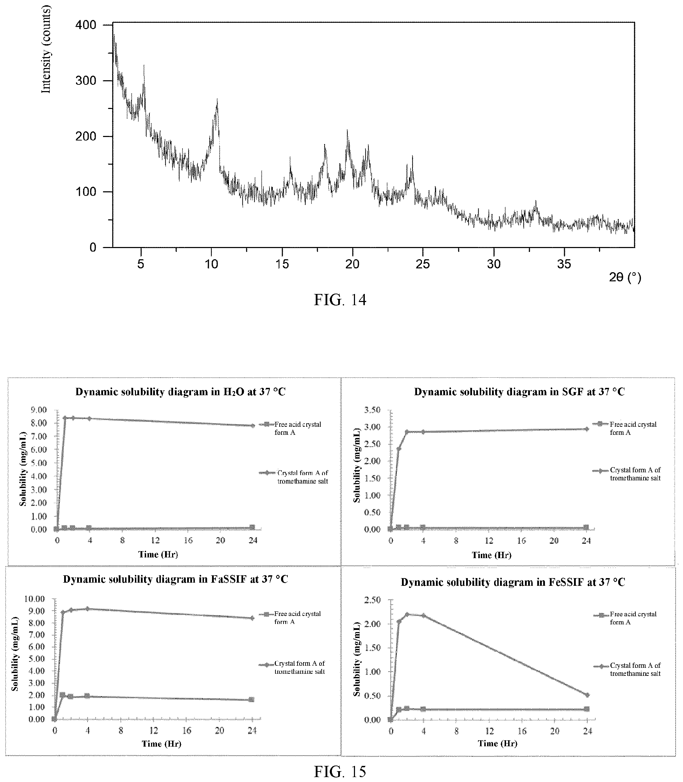

In some embodiments, the present disclosure provides crystal form A of the lysine salt of the compound of formula I, and the crystal form A of the lysine salt has an X-ray powder diffraction pattern comprising diffraction peaks at 2θ±0.2° diffraction angles of 5.12, 10.44, 15.56, 18.07, 19.61, and 21.10; further, the crystal form A of the lysine salt has an X-ray powder diffraction pattern comprising diffraction peaks at 2θ±0.2° diffraction angles of 5.12, 10.44, 15.56, 18.07, 19.61, 21.10, 24.22, and 32.97; furthermore, the crystal form A of the lysine salt has an XRPD pattern substantially shown in FIG. 14.

In another aspect, the present disclosure provides a preparation method for the free acid crystal form A of the compound of formula I, which includes the following methods.

Method 1 comprises: adding the compound of formula I into an organic solvent I, and volatilizing at room temperature, wherein the organic solvent I may be selected from one or a mixture of more of ethyl acetate, dichloromethane, methyl *tert*-butyl ether, isopropanol, and water.

Method 2 comprises: performing anti-solvent addition crystallization on the compound of formula I in an organic solvent II; if no solid is precipitated by stirring, adding an anti-solvent into the system, wherein the organic solvent II may be selected from one or a mixture of more of methanol, methyl ethyl ketone, isopropyl acetate, tetrahydrofuran, methyl *tert*-butyl ether, and dimethylacetamide; the anti-solvent may be selected from one or a mixture of more of water, isopropyl ether, toluene, *m-*xylene, 4-cymene, *n*-pentane, *n*-heptane, cyclohexane, and methylcyclohexane.

Method 3 comprises: suspending and stirring the compound of formula I in an organic solvent III at room temperature for crystallization, wherein the organic solvent III may be selected from one or a mixture of more of *n*-pentane, toluene, *m*-xylene, isopropyl ether, *n*-hexane, cyclohexane, methylcyclohexane, water, methanol, *N,N*-dimethylformamide, 2-methyltetrahydrofuran, acetone, methyl acetate, dichloromethane, and acetonitrile.

Method 4 comprises: suspending and stirring the compound of formula I in an organic solvent IV at 40-60 °C (e.g., 50 °C) for crystallization, wherein the organic solvent IV may be selected from one or a mixture of more of methylcyclohexane, cumene, water, 1,4-dioxane, dimethylacetamide, tetrahydrofuran, n-hexane, 2-methyltetrahydrofuran, n-pentane, methyl ethyl ketone, isopropyl acetate, toluene, isobutanol, trichloromethane, and *m*-xylene.

Method 5 comprises: performing wet grinding on the compound of formula I in an organic solvent V for crystallization, wherein the organic solvent V may be selected from one or a mixture of more of ethanol, dichloromethane, ethyl acetate, and tetrahydrofuran.

In another aspect, the present disclosure provides a preparation method for the free acid crystal form B of the compound of formula I, comprising the following steps: dissolving the free acid crystal form A of the compound of formula I in an organic solvent B 1, and then performing gas-liquid diffusion in an atmosphere of an organic solvent B2, wherein the organic solvent B 1 is selected from ketones, such as methyl ethyl ketone, methyl isopropyl ketone, acetone, diethyl ketone, dipropyl ketone, diisopropyl ketone, dibutyl ketone, and diisobutyl ketone; the organic solvent B2 is selected from alkane organic compounds, preferably C1-C7 alkane organic compounds, for example, it is selected from *n*-pentane, *n*-heptane, and cyclohexane. In some embodiments, the free acid crystal form A of the compound of formula I is dissolved in methyl isobutyl ketone (MIBK), and then the gas-liquid diffusion is performed in an *n*-pentane atmosphere to give the free acid crystal form B of the compound of formula I.

The present disclosure further provides a preparation method for the pharmaceutically acceptable salt of the compound of formula I, comprising the following step: mixing the compound of formula I with a salt-forming reagent (such as a corresponding base) in a suitable solvent. In some embodiments, the solvent is selected from one or a mixture of more of ethanol, heptane, ethyl acetate, MTBE, acetonitrile, water, and acetone.

In yet another aspect, the present disclosure provides a pharmaceutical composition comprising one or more of the free acid crystal forms (e.g., free acid crystal form A and free acid crystal form B) of the compound of formula I and the pharmaceutically acceptable salts (including crystal forms thereof) of the compound of formula I.

In yet another aspect, the present disclosure provides use of the free acid crystal form (e.g., free acid crystal form A or free acid crystal form B) of the compound of formula I, the pharmaceutically acceptable salt (including the crystal form thereof) of the compound of formula I, or the pharmaceutical composition for the manufacturing of a medicament for treating or preventing a disease associated with EP4.

According to an embodiment of the present disclosure, the disease associated with EP4 includes at least one selected from the following: inflammatory diseases, pain, cancers, metabolic diseases, and urinary system diseases; the inflammatory disease includes at least one selected from the following: arthritis and rheumatoid arthritis; the pain includes osteoarthritis pain and pain caused by endometriosis.

According to an embodiment of the present disclosure, the free acid crystal form (e.g., free acid crystal form A or free acid crystal form B) of the compound of formula I, the pharmaceutically acceptable salt (including the crystal form thereof) of the compound of formula I, or the pharmaceutical composition may be administered in combination with radiotherapy and/or antibody therapy, wherein the antibody therapy is selected from one or a combination of CTLA4 antibody therapy, PDL1 antibody therapy, and PD1 antibody therapy.

According to embodiments of the present disclosure, the cancer includes a solid cancer. In some embodiments, the cancer includes breast cancer, cervical cancer, colorectal cancer, endometrial cancer, glioblastoma, head and neck cancer, kidney cancer, liver cancer, lung cancer, medulloblastoma, ovarian cancer, pancreatic cancer, prostate cancer, skin cancer, and urinary tract cancer.

According to embodiments of the present disclosure, the metabolic disease includes diabetes, and the urinary system disease includes overactive bladder.

According to embodiments of the present disclosure, a better and more effective clinical therapeutic medicament or regimen may be provided to a patient in need thereof by using the free acid crystal form (e.g., free acid crystal form A or free acid crystal form B) of the compound of formula I, the pharmaceutically acceptable salt (including the crystal form thereof) of the compound of formula I, or the pharmaceutical composition of the present disclosure.

The present disclosure also relates to a method for treating a disease associated with EP4, the method comprising administering to a patient a therapeutically effective dose of a pharmaceutical formulation comprising the free acid crystal form (e.g., free acid crystal form A or free acid crystal form B) of the compound of formula I, the pharmaceutically acceptable salt (including the crystal form thereof) of the compound of formula I, or the pharmaceutical composition.

### Definitions and explanations of terms

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification.

Unless otherwise stated, a numerical range set forth in the description and claims shall be construed as at least including each specific integer value within the range. For example, two or more represent 2, 3, 4, 5, 6, 7, 8, 9, 10 or more. When certain numerical ranges are defined or understood as "numbers", it shall be construed as including both endpoints of the range, each integer within the range, and each decimal within the range. For example, "a number of 0-10" shall be construed as including not only each of integers 0, 1, 2, 3, 4, 5, 6, 7, 8, 9, and 10, but also at least the sums of each integer and 0.1, 0.2, 0.3, 0.4, 0.5, 0.6, 0.7, 0.8, or 0.9.

When "about" numerical value is recited in the specification and claims, it shall be construed as including the numerical value itself, as well as numerical values within a range around the numerical value that is acceptable in the art, for example, numerical values within the range of ±15% of the numerical value, numerical values within the range of ±10% of the numerical value and numerical values within the range of ±5% of the numerical value. For example, about 10 represents that it includes a value within the range of 10:1:1.5, i.e., within the range of 8.5 to 11.5; a value within the range of 10:1:1.0, i.e., within the range of 9.0 to 11.0; and a value within the range of 10±0.5, i.e., within the range of 9.5 to 10.5.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians or other clinicians are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition.(i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms).

The term "pharmaceutically acceptable" means that a formula component or an active ingredient does not unduly adversely affect a general therapeutic target's health.

The term "pharmaceutically acceptable excipient or carrier" refers to one or more compatible solid or liquid fillers or gel substances which are suitable for human use and must be of sufficient purity and sufficiently low toxicity. "Compatible" means that the components of the composition are capable of intermixing with the compound of the present disclosure and with each other, without significantly diminishing the pharmaceutical efficacy of the compound. Examples of pharmaceutically acceptable excipients or carriers are cellulose and its derivatives (e.g., sodium carboxymethylcellulose, sodium ethylcellulose, cellulose acetate and the like), gelatin, talc, solid lubricants (e.g., stearic acid and magnesium stearate), calcium sulfate, vegetable oil (e.g., soybean oil, sesame oil, peanut oil, olive oil and the like), polyols (e.g., propylene glycol, glycerol, mannitol, sorbitol and the like), emulsifiers, wetting agents (e.g., sodium dodecyl sulfate), colorants, flavoring agents, stabilizers, antioxidants, preservatives, pyrogen-free water, etc.

### Beneficial Effects

1) The present disclosure provides crystal forms of a compound of formula I, which have good medicinal properties, wherein free acid crystal form A has relatively low hygroscopicity and good physicochemical stability, and is beneficial to the storage and quality stability of the medicament and further druggability.
2) The present disclosure screens and obtains pharmaceutically acceptable salts of the compound of formula I by optimization tests, and further obtains crystal form products of the salts, such as crystal form A of a tromethamine salt, crystal form A of a diethylamine salt and crystal form A of a lysine salt, wherein the crystal form A of the tromethamine salt has relatively low hygroscopicity, good physicochemical stability, higher solubility in biological vehicles, and better druggability value.

### BRIEF DESCRIPTION OF THE DRAWING

FIG. 1 is an XRPD pattern of free acid crystal form A of a compound of formula I;
FIG. 2 is a TGA profile of the free acid crystal form A of the compound of formula I;

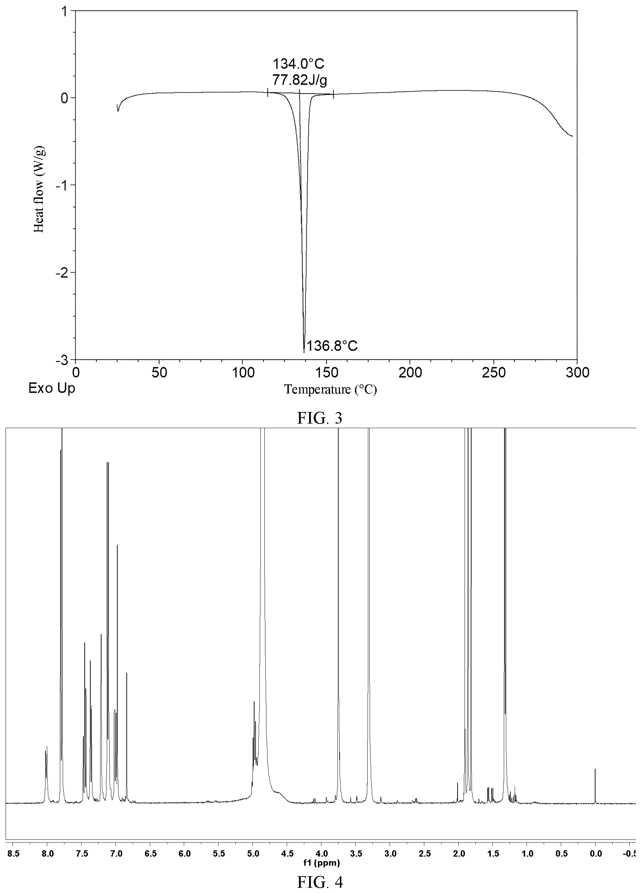

FIG. 3 is a DSC profile of the free acid crystal form A of the compound of formula I;
FIG. 4 is a ¹H NMR spectrum of the free acid crystal form A of the compound of formula I;
FIG. 5 is an XRPD pattern of free acid crystal form B of the compound of formula I;
FIG. 6 is an XRPD pattern of crystal form A of a tromethamine salt of the compound of formula I;
FIG. 7 is a TGA profile of the crystal form A of the tromethamine salt of the compound of formula I;
FIG. 8 is a DSC profile of the crystal form A of the tromethamine salt of the compound of formula I;
FIG. 9 is a ¹H NMR spectrum of the crystal form A of the tromethamine salt of the compound of formula I;
FIG. 10 is an XRPD pattern of crystal form A of a diethylamine salt of the compound of formula I;
FIG. 11 is a TGA profile of the crystal form A of the diethylamine salt of the compound of formula I;
FIG. 12 is a DSC profile of the crystal form A of the diethylamine salt of the compound of formula I;
FIG. 13 is a ¹H NMR spectrum of the crystal form A of the diethylamine salt of the compound of formula I;
FIG. 14 is an XRPD pattern of crystal form A of a lysine salt of the compound of formula I;
FIG. 15 is a dynamic solubility curve at 37 °C;

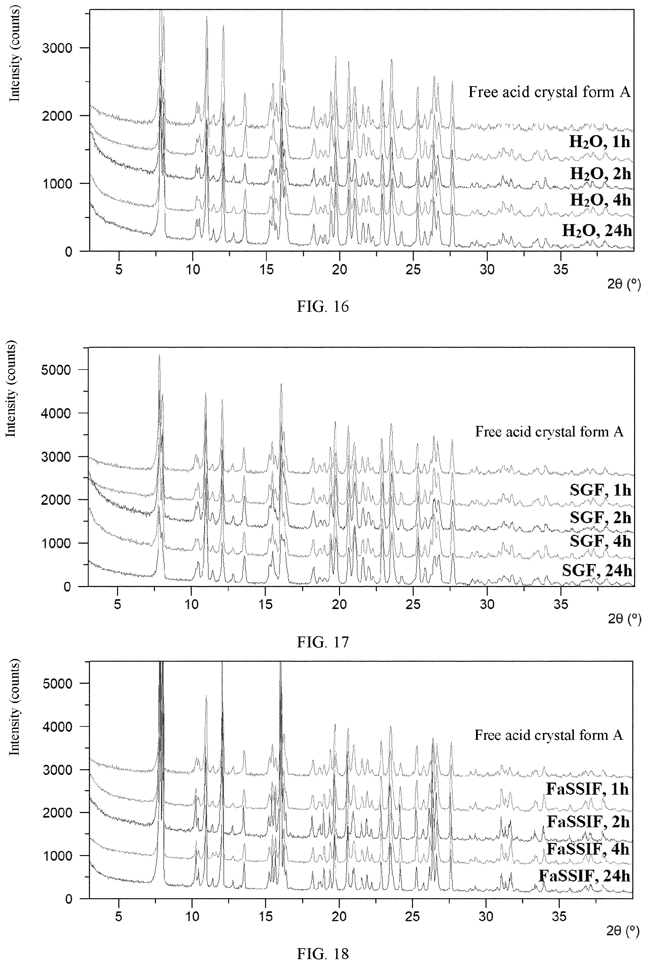

FIG. 16 is an XRPD overlay of solubility samples of the free acid crystal form A in H₂O;
FIG. 17 is an XRPD overlay of solubility samples of the free acid crystal form A in SGF;
FIG. 18 is an XRPD overlay of solubility samples of the free acid crystal form A in FaSSIF;

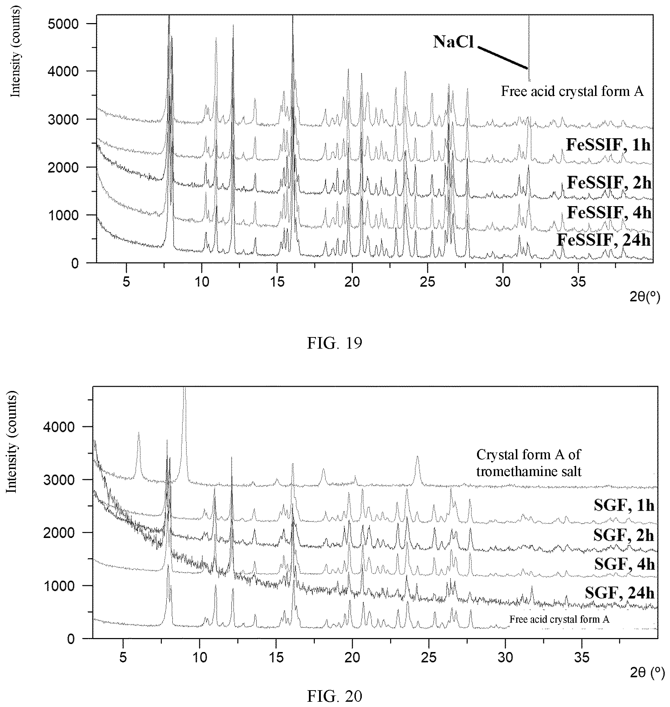

FIG. 19 is an XRPD overlay of solubility samples of the free acid crystal form A in FeSSIF;
FIG. 20 is an XRPD overlay of solubility samples of the crystal form A of the tromethamine salt in SGF;

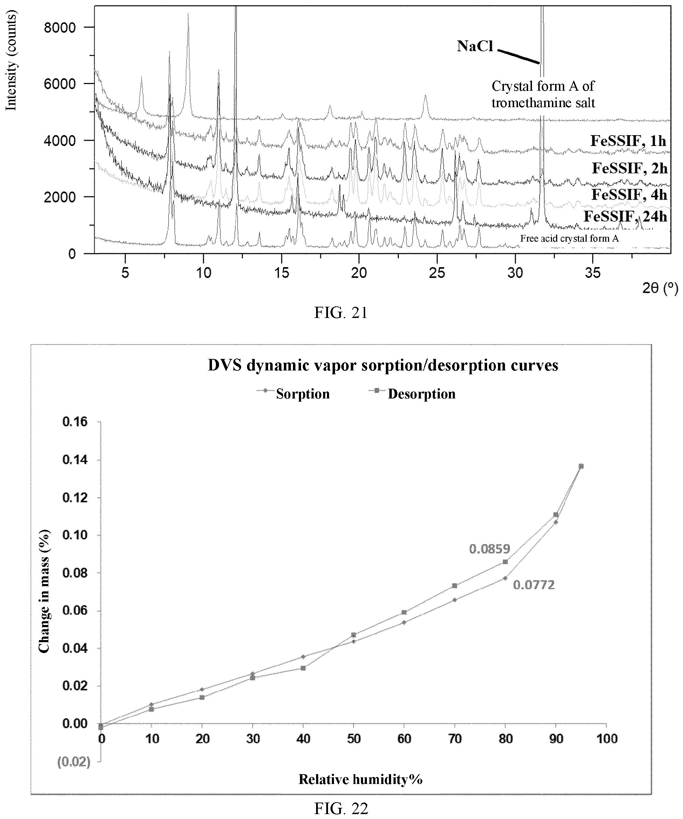

FIG. 21 is an XRPD overlay of solubility samples of the crystal form A of the tromethamine salt in FeSSIF;
FIG. 22 is a DVS profile of the free acid crystal form A;

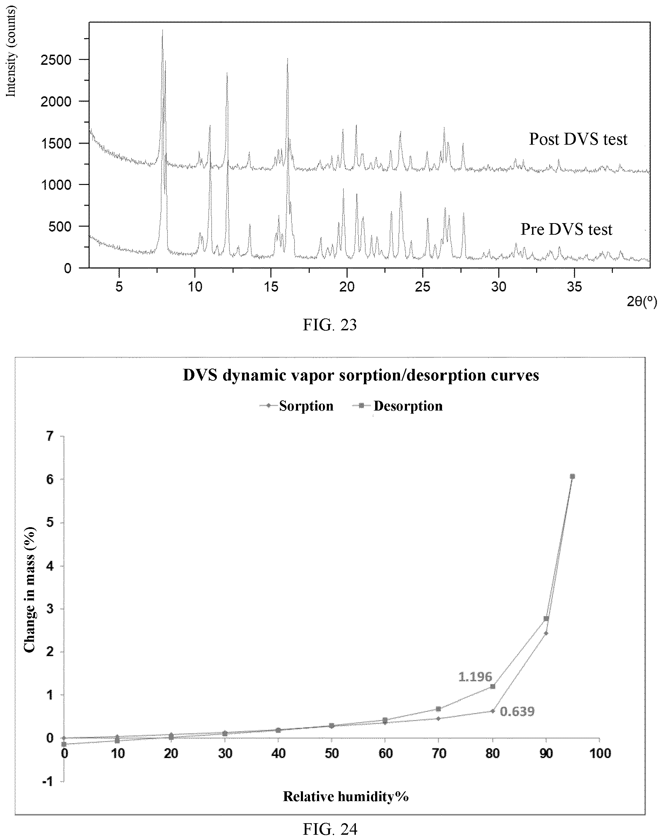

FIG. 23 is an XRPD overlay of the free acid crystal form A before and after DVS test;
FIG. 24 is a DVS profile of the crystal form A of the tromethamine salt;

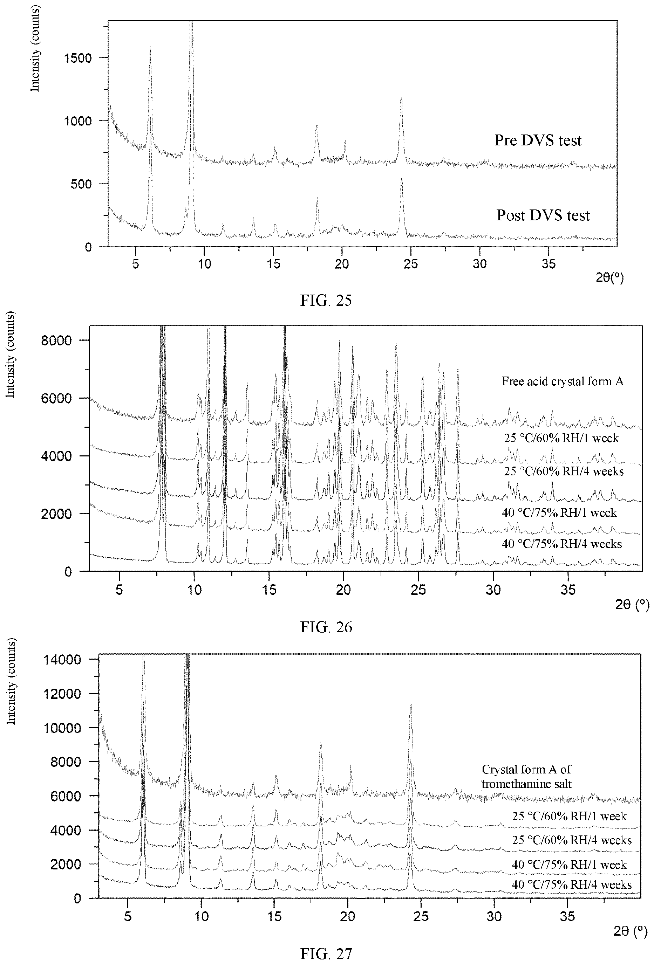

FIG. 25 is an XRPD overlay of the crystal form A of the tromethamine salt before and after DVS test;
FIG. 26 is an XRPD overlay of samples for evaluating the stability of the free acid crystal form A; and
FIG. 27 is an XRPD overlay of samples for evaluating the stability of the crystal form A of the tromethamine salt.

### DETAILED DESCRIPTION

The technical solutions of the present disclosure will be further described in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products, or can be prepared by using known methods.

The instruments and detection methods adopted by the present disclosure are as follows:

### I. X-ray powder diffraction (XRPD)

XRPD patterns are acquired on an X-ray powder diffraction analyzer manufactured by PANalytacal, and the scanning parameters are shown in Table A-1 below.

**Table A-1. XRPD test parameters**

| **Model** | **X' Pert3/Empyrean** |
|---|---|
| X-ray | Cu, Kα, Kα1 (Å):1.540598, Kα2 (Å):1.544426 |
| | Kα2/Kα1 intensity ratio:0.50 |
| X-ray light tube settings | 45 kV, 40 mA |
| Divergence slit | 1/8° |
| Scanning mode | Continuous |
| Scanning range (°2Theta) | 3-40 |
| Scanning time of each step (s) | 46.7 |
| Scanning step length (°2Theta) | 0.0263 |
| Test time | ∼5 min |
| Model | X' Pert3 |

### II. Thermogravimetric analysis (TGA) and differential scanning calorimetry (DSC)

TGA and DSC profiles are acquired on a TA Q5000/5500 thermogravimetric analyzer and a TA Q2000/2500 differential scanning calorimeter, respectively. The test parameters are listed in Table A-2 below.

**Table A-2. DSC and TGA test parameters**

| **Parameter** | **TGA** | **DSC** |
|---|---|---|
| Method | Linear heating | Linear heating |
| Sample tray | Aluminum pan, open | Aluminum pan, gland/non-gland |
| Temperature range | Room temperature-setting endpoint temperature | 25 °C-setting endpoint temperature |
| Purging speed (°C/min) | 10 | 10 |
| Protective gas | Nitrogen | Nitrogen |

### III. Dynamic vapor sorption (DVS)

Dynamic vapor sorption (DVS) curves are acquired on a DVS IntrInsic in Surface Measurement Systems (SMS). The relative humidity at 25 °C is corrected with the deliquescence points of LiCl, Mg(NO₃)₂, and KCl. The DVS test parameters are listed in Table A-3 below.

**Table A-3. DVS test parameters**

| **Parameter** | **Set value** |
|---|---|
| Temperature | 25 °C |
| Sample amount | 10-20 mg |
| Protective gas and flow | N₂, 200 mL/min |
| dm/dt | 0.002%/min |
| Minimum dm/dt equilibration time | 10 min |
| Maximum equilibrium of time | 180 min |
| RH range | 0%RH-95%RH |
| RH gradient | 10%RH (0%RH ∼ 90%RH & 90%RH ∼ 0%RH) |
| | 5%RH (90%RH ∼ 95%RH & 95%RH ∼ 90%RH) |

### IV. Liquid-state nuclear magnetic resonance (¹H NMR)

Liquid-state nuclear magnetic resonance spectra are acquired on a Bruker 400M nuclear magnetic resonance spectrometer with CD₃OD as a solvent.

### V. High-performance liquid chromatography (HPLC)

In the test, the purity, dynamic solubility and stability are tested by an Agilent 1260 high-performance liquid chromatograph. The analysis conditions are shown in Table A-4 below.

**Table A-4. High-performance liquid chromatography test conditions**

| | | | | |
|---|---|---|---|---|
| **Liquid chromatograph** | **Agilent 1260 detector** | | | |
| Chromatographic column | ACE 3 C18-AR, 4.6 mm/150 mm/3 µm | | | |
| Mobile phase | A: 0.1% FA in H₂O | | | |
| | B: Acetonitrile | | | |
| Elution gradient | Purity | | Solubility | |
| | **Time (min)** | **%B** | **Time (min)** | **%B** |
| | 0.0 | 5 | 0.0 | 10 |
| | 10.0 | 5 | 5.0 | 90 |
| | 12.0 | 95 | 6.0 | 90 |
| | 13.0 | 95 | 6.1 | 10 |
| | 13.1 | 5 | 8.0 | 10 |
| | 15.0 | 5 | -- | -- |
| Run time | 15 min | | 8 min | |
| Flow rate of mobile phase | 1.5 mL/min | | | |
| Injection volume | 5 µL | | | |
| Detection wavelength | UV at 254 nm | | | |
| Column temperature | 40 °C | | | |
| Injector temperature | RT | | | |
| Diluent | Acetonitrile /H₂O=1:1 (v/v) | | | |

The reagents used in the present disclosure are shown in Table A-5 below.

**Table A-5. Names of solvents used in the test**

| **Abbreviation** | **Name** | **Abbreviation** | **Name** |
|---|---|---|---|
| MeOH | Methanol | 1,4-dioxane | 1,4-dioxane |
| EtOH | Ethanol | ACN | Acetonitrile |
| IPA | Isopropanol | DCM | Dichloromethane |
| Acetone | Acetone | CHCl₃ | Trichloromethane |
| MIBK | Methyl isobutyl ketone | Toluene | Toluene |
| EtOAc | Ethyl acetate | n-Heptane | n-Heptane |
| IPAc | Isopropyl acetate | DMSO | Dimethyl sulfoxide |
| MTBE | Methyl tert-butyl ether | DMAc | N,N-Dimethylacetamide |
| THF | Tetrahydrofuran | NMP | N-Methylpyrrolidone |
| 2-MeTHF | 2-Methyltetrahydrofuran | H₂O | Water |
| CPME | Cyclopentyl methyl ether | n-Pentane | n-Pentane |
| m-Xylene | m-Xylene | Cyclohexane | Cyclohexane |
| p-Cymene | p-Cymene | MEK | 2-Butanone |
| n-Hexane | n-Hexane | Isobutanol | Isobutanol |
| Methyl butyl ether | Methyl butyl ether | Cumene | Cumene |
| Methylcyclohexane | Methylcyclohexane | Anisole | Anisole |
| Butyl acetate | Butyl acetate | / | / |

### Example 1. Preparation of Intermediate A

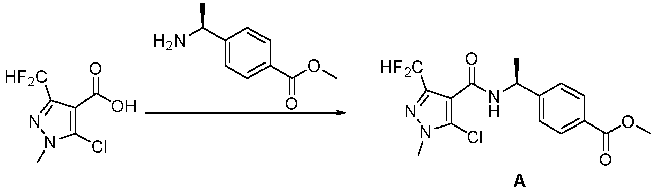

At room temperature, the starting material 5-chloro-3-(difluoromethyl)-1-methyl-1*H*-pyrazole-4-carboxylic acid (5 g, 23.8 mmol) (synthesized with reference to patent application WO2011151369A1) was added to DCM (dichloromethane) (200 mL), methyl (*S*)-4-(1-aminoethyl)benzoate (5.1 g, 28.6 mmol), HATU (O-(7-azabenzotriazol-1-yl)-*N,N,N',N'-*tetramethylurea hexafluorophosphate) (10.9 g, 28.6 mmol), and DIPEA (*N,N*-diisopropylethylamine) (4.6 g, 35.7 mmol) were added, and the mixture was stirred at room temperature for 16 h. Water (200 mL) was added, the mixed solution was extracted with DCM (50 mL × 3), and liquid separation was performed. The organic phases were combined, dried over anhydrous sodium sulfate, filtered, and concentrated. The residue was separated and purified by a silica gel column (petroleum ether:ethyl acetate (V/V) = 3:1) to give methyl (*S*)-4-(1-(5-chloro-3-(difluoromethyl)-1-methyl-1*H*-pyrazole-4-carboxamide)ethyl)benzoate (intermediate A) as a white solid (5.6 g, 63.3% yield). LCMS (ESI) m/z: 372.5 [M+H]⁺.

### Example 2. Preparation of Compound of Formula I

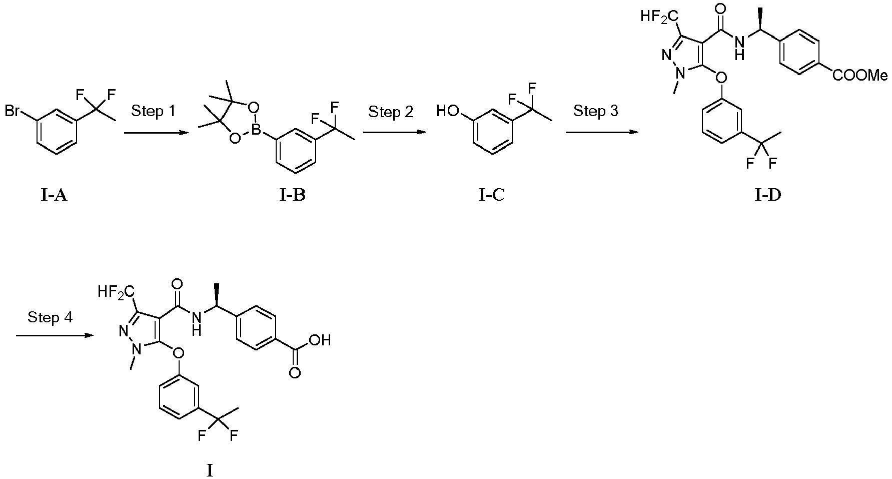

### Step 1: 2-(3-(1, 1-difluoroethyl)phenyl)pinacol boronate (compound I-B)

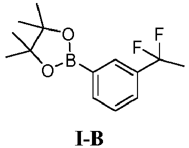

1-Bromo-3-(1,1-difluoroethyl)benzene (800 mg, 3.62 mmol) was added to 1,4-dioxane (30 mL) at room temperature, bis(pinacolato)diboron (17.0 g, 156.3 mmol), copper(I) iodide (2.5 g, 13.0 mmol), L-proline (2.76 g, 10.86 mmol), potassium acetate (710 mg, 7.24 mmol), and [1,1-bis(diphenylphosphino)ferrocene]palladium(II) dichloride (295 mg, 0.36 mmol) were added, and the mixture was heated to 90 °C under nitrogen atmosphere and stirred for 16 h. The mixture was cooled to room temperature, and water (200 mL) was added for dilution. The mixed solution was extracted with dichloromethane (80 mL × 3), and liquid separation was performed. The organic phases were combined, dried over anhydrous sodium sulfate, filtered, and concentrated. The residue was separated and purified by a silica gel column (pure petroleum ether) to give crude 2-(3-(1,1-difluoroethyl)phenyl)pinacol boronate (**compound I-B**) as a colorless liquid (900 mg, 92.7% yield).

### Step 2: 3-(1,1-difluoroethyl)phenol (compound I-C)

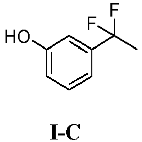

2-(3-(1,1-Difluoroethyl)phenyl)pinacol boronate (**compound I-B**) (900 mg, 3.36 mmol) was added to THF (15 mL) and water (15 mL), and sodium perborate monohydrate (1.01 g, 10.07 mmol) was added, and the mixture was stirred at room temperature for 16 h. Water (200 mL) was added for dilution, the mixed solution was extracted with DCM (50 mL × 3), and liquid separation was performed. The organic phases were combined, dried over anhydrous sodium sulfate, filtered, and concentrated. The residue was separated and purified by a silica gel column (petroleum ether:ethyl acetate (V/V) = 8:1) to give 3-(1,1-difluoroethyl)phenol (**compound I-C**) as a colorless liquid (280 mg, 52.7% yield).

### Step 3: methyl (S)-4-(1-(5-(3-(1,1-difluoroethyl)phenoxy)-3-(difluoromethyl)-1-methyl-1H-pyrazole-4-carboxamide)ethyl)benzoate (compound I-D)

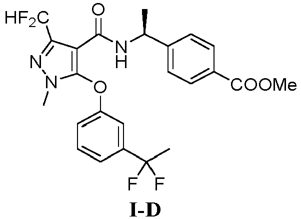

The compound methyl (*S*)-4-(1-(5-chloro-3-(difluoromethyl)-1-methyl-1*H*-pyrazole-4-carboxamide)ethyl)benzoate (**intermediate A**) (650 mg, 1.75 mmol) was added to DMF (12 mL) at room temperature, 3-(1,1-difluoroethyl)phenol **(I-C)** (360 mg, 2.27 mmol) and potassium hydroxide (147 mg, 2.62 mmol) were added, and the mixture was heated to 120 °C and stirred for 2 h. The mixture was cooled to room temperature, and water (200 mL) was added for dilution. The mixed solution was extracted with ethyl acetate (80 mL × 3), and liquid separation was performed. The organic phases were combined, dried over anhydrous sodium sulfate, filtered, and concentrated to give crude methyl (*S*)-4-(1-(5-(3-(1,1-difluoroethyl)phenoxy)-3-(difluoromethyl)-1-methyl-1*H-*pyrazole-4-carboxamide)ethyl)benzoate (**compound I-D**) as a colorless liquid (1.2 g, crude).

LCMS (ESI) m/z: 494.6 [M+H]⁺.

### Step 4: (S)-4-(1-(5-(3-(1,1-difluoroethyl)phenoxy)-3-(difluoromethyl)-1-methyl-1H-pyrazole-4-carboxamide)ethyl)benzoic acid (compound I)

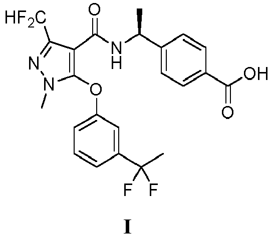

The starting material methyl (*S*)-4-(1-(5-(3-(1,1-difluoroethyl)phenoxy)-3-(difluoromethyl)-1-methyl-1*H*-pyrazole-4-carboxamide)ethyl)benzoate (**compound I-D**) (1.0 g, 2.03 mmol) was added to THF (5 mL) at room temperature, water (4 mL) and lithium hydroxide monohydrate (340 mg, 8.11 mmol) were added, and the mixture was stirred at room temperature for 16 h. The reaction liquid was concentrated to give (*S*)-4-(1-(5-(3-(1,1-difluoroethyl)phenoxy)-3-(difluoromethyl)-1-methyl-1*H-*pyrazole-4-carboxamide)ethyl)benzoic acid (**compound I**) as a white solid (88 mg, 7.9% yield).

LCMS (ESI) m/z: 480.5 [M+H]⁺.

¹H NMR (400m Hz, DMSO-d₆) δ12.8 (s, 1H), 8.10 (d, 1H), 7.71 (d, 2H), 7.53 (t, 1H), 7.47 (d, 1H), 7.27 (d, 1H), 7.11 (t, 1H), 7.11 (d, 2H), 7.07 (dd, 1H), 4.88 (t, 1H), 3.74 (s, 3H), 1.96 (t, 3H), 1.96 (d, 3H).

### Example 3. Preparation of Free Acid Crystal Form A of Compound of Formula I

The compound of formula I prepared according to the method in Example 2 was dissolved in a mixed solvent of dichloromethane and *n*-hexane (1:10, v/v). The mixture was concentrated to give free acid crystal form A of the compound of formula I.

The XRPD pattern of the obtained free acid crystal form A of the compound of formula I is shown in FIG. 1. The TGA/DSC results (FIGs. 2-3) show a weight loss of 0.84% when the sample was heated to 150 °C and an endothermic peak at 136.8±3 °C. The ¹H NMR result of the free acid crystal form A is shown in FIG. 4. Given that the free acid crystal form A showed a relatively low weight loss in TGA weight loss and had only a single melting endothermic signal in DSC, it is presumed to be an anhydrous crystal form.

The XRPD analysis data of the free acid crystal form A of the compound of formula I obtained are shown in Table 3-1 below:

**Table 3-1. XRPD diffraction peak data of free acid crystal form A**

| Peak number | 2θ [°] | Relative intensity [%] |
|---|---|---|
| 1 | 7.8776 | 100.00 |
| 2 | 8.0779 | 66.26 |
| 3 | 10.3144 | 14.36 |
| 4 | 10.4871 | 11.43 |
| 5 | 10.9994 | 67.36 |
| 6 | 11.4644 | 6.69 |
| 7 | 12.1311 | 60.62 |
| 8 | 12.8221 | 5.25 |
| 9 | 13.5910 | 19.59 |
| 10 | 15.2935 | 14.97 |
| 11 | 15.4979 | 27.14 |
| 12 | 15.7226 | 15.29 |
| 13 | 16.1047 | 79.00 |
| 14 | 18.2731 | 13.24 |
| 15 | 18.7048 | 6.58 |
| 16 | 19.0384 | 8.94 |
| 17 | 19.4405 | 22.48 |
| 18 | 19.7525 | 44.87 |
| 19 | 20.6453 | 41.55 |
| 20 | 21.0382 | 25.61 |
| 21 | 21.6162 | 13.84 |
| 22 | 21.9700 | 13.66 |
| 23 | 22.2681 | 5.32 |
| 24 | 22.9183 | 29.82 |
| 25 | 23.5279 | 42.75 |
| 26 | 24.2241 | 11.46 |
| 27 | 25.3202 | 25.97 |
| 28 | 25.8127 | 8.64 |
| 29 | 26.2110 | 12.12 |
| 30 | 26.4374 | 33.22 |
| 31 | 26.6926 | 27.62 |
| 32 | 27.6732 | 28.44 |
| 33 | 28.9891 | 3.46 |
| 34 | 29.3504 | 6.19 |
| 35 | 30.1661 | 2.42 |
| 36 | 31.1181 | 10.41 |
| 37 | 31.6472 | 7.63 |
| 38 | 32.1840 | 3.10 |
| 39 | 33.4412 | 4.76 |
| 40 | 33.9719 | 7.86 |
| 41 | 35.7828 | 2.63 |
| 42 | 36.8424 | 4.71 |
| 43 | 37.1821 | 5.29 |
| 44 | 37.9932 | 5.63 |

### Example 4. Preparation of Free Acid Crystal Form B of Compound of Formula I

The free acid crystal form A was dissolved in MIBK (methyl isobutyl ketone), and then the mixture was subjected to gas-liquid diffusion in an n-pentane atmosphere to obtain free acid crystal form B (XRPD pattern thereof shown in FIG. 5).

The free acid crystal form B was converted into the free acid crystal form A after being dried at room temperature.

The XRPD analysis data of the free acid crystal form B of the compound of formula I obtained are shown in Table 4-1 below:

**Table 4-1. XRPD diffraction peak data of free acid crystal form B**

| Peak number | 2θ [°] | Relative intensity [%] |
|---|---|---|
| 1 | 5.5566 | 16.73 |
| 2 | 5.9962 | 14.18 |
| 3 | 7.3831 | 40.61 |
| 4 | 8.2693 | 9.80 |
| 5 | 8.9073 | 100.00 |
| 6 | 11.0705 | 29.81 |
| 7 | 11.5756 | 19.24 |
| 8 | 12.0123 | 79.61 |
| 9 | 13.5818 | 18.08 |
| 10 | 14.1578 | 14.08 |
| 11 | 14.7785 | 27.31 |
| 12 | 15.7341 | 6.80 |
| 13 | 17.8520 | 26.81 |
| 14 | 18.5175 | 22.43 |
| 15 | 19.3839 | 36.89 |
| 16 | 22.3300 | 26.08 |
| 17 | 23.0493 | 60.19 |
| 18 | 24.6436 | 11.03 |
| 19 | 26.0076 | 29.65 |
| 20 | 26.7582 | 28.55 |

### Example 5. Preparation and Screening of Salt Forms of Compound of Formula I

About 20 mg of an initial free acid crystal form A sample and an equimolar amount of a salt-forming formulation (i.e., a base for forming a salt with a free acid) were added into an HPLC vial, and 0.5 mL of a solvent was added and mixed to give a suspension. The salt-forming formulation was mixed with the initial sample after being diluted with a corresponding solvent. After the mixture was suspended and stirred at room temperature for about 3 days, the solid was separated out by centrifugation and dried under vacuum at room temperature overnight. For a gelling system at room temperature, the mixture was transferred to 50 °C-5 °C and subjected to cyclic heating and cooling to induce polymorphic transition. For a clarifying system at room temperature, the mixture was transferred to 5 °C or -20 °C and stirred to induce crystallization. The XRPD characterization results of the resulting solids showed that a total of 3 salt forms were obtained in the salt form screening test (as shown in Table 5-1 below).

**Table 5-1. Summary of salt form screening test results**

| Serial number | Base^{#} | A EtOH/n-heptane (1:3, v/v) | B EtOAc | C MTBE | D ACN/H₂O (1:1, v/v) | E Acetone/n-heptane (1:2, v/v) |
|---|---|---|---|---|---|---|
| 0 | Blank | Free acid crystal form A | Free acid crystal form A* | Free acid crystal form A | Free acid crystal form A* | Free acid crystal form A* |
| 1 | Sodium hydroxide | Amorphous form (colloidal) * | Amorphous form (colloidal) * | Amorphous form (colloidal) * * | Amorphous form (colloidal) * | Gelling* |
| 2 | Potassium hydroxide | Amorphous form (colloidal) * | Amorphous form* | Amorphous form | Gelling* | Gelling* |
| 3 | L-arginine | Amorphous form* | Gelling* | L-arginine | Amorphous form* | Amorphous form* |
| 4 | Calcium hydroxide | Free acid crystal form A+ Calcium hydroxide | Calcium hydroxide | Free acid crystal form A+ Calcium hydroxide | Calcium hydroxide | Calcium hydroxide |
| 5 | Magnesium hydroxide | Magnesium hydroxide (colloidal) | Magnesium hydroxide | Free acid crystal form A+ Magnesium hydroxide | Magnesium hydroxide* | Magnesium hydroxide* |
| 6 | Choline | Free crystal form A* | Oil forming* | Free acid crystal form A (colloidal)^ | Amorphous form* | Free acid crystal form A^ |
| 7 | Aqueous ammonia | Amorphous form (oil forming)* | Oil forming* | Free acid crystal form A^ | Gelling* | Gelling* |
| 8 | Meglumine | Amorphous form (colloidal) * | Meglumine | Meglumine (colloidal) | Gelling* | Gelling* |
| 9 | Lysine | Free acid crystal form A (colloidal) | Lysine salt Crystal form A | Free acid crystal form A | Free acid crystal form A* | Free acid crystal form A+ multimodal* |
| 10 | Tromethamine | Amorphous form* | Tromethamine salt Crystal form A | Tromethamine salt Crystal form A | Gelling* | Tromethamine salt Crystal form A** |
| 11 | Betaine | Free acid crystal form A | Betaine | Free acid crystal form A | Betaine* | Free acid crystal form A^ |
| 12 | Diethylamine | Amorphous form (oil forming)* | Oil forming* | Diethylamine salt Crystal form A | Gelling* | Amorphous form (oil forming)* |
| 13 | Zinc hydroxide | Free acid crystal form A+ Zinc hydroxide | Zinc hydroxide | Free acid crystal form A+ Zinc hydroxide | Zinc hydroxide | Weak crystallinity (colloidal) |
| 14 | Monoethanolamine | Oil forming* | Oil forming* | Amorphous form (oil forming)* | Gelling* | Amorphous form (oil forming)* |
| 15 | Caffeine | Free acid crystal form A+ Caffeine | Caffeine | Free acid crystal form A+ Caffeine | Caffeine * | Caffeine |
| 16 | Urea | Free acid crystal form A | Free acid crystal form A+ Urea* | Free acid crystal form A+ Urea | Urea (colloidal) * | Urea (colloidal) * |
| 17 | Nicotinamide | Free acid crystal form A | Gelling* | Free acid crystal form A+ Nicotinamide | Free acid crystal form A+ Nicotinamide (colloidal) * | Nicotinamide (colloidal) |
| 18 | Isonicotine | Free acid crystal form A | Isonicotine (colloidal) * | Free acid crystal form A+ Isonicotine | Isonicotine* | Isonicotine (colloidal) |
| 19 | Diethanolamine | Oil forming* | Oil forming* | Amorphous form (oil forming)* | Oil forming* | Amorphous form (oil forming)* |
| 20 | Imidazole | Oil forming* | Oil forming* | Amorphous form (oil forming)* | Oil forming* | Amorphous form (oil forming)* |

| | | | | | | |
|---|---|---|---|---|---|---|
| ^{#}: the feeding molar ratio of acid/base was 1:1. **: no solid precipitation was observed in the sample after 2 days of slurrying; then the sample was transferred to temperature cycling, and solid precipitation was observed. ^: no solid precipitation was observed in the sample after 2 days of slurrying; then the sample was transferred to temperature cycling and placed at -20 °C, and no solid was observed; then 0.5 mL of n-heptane (columns A and E)/MTBE (column B) was added, the mixture was slurried at room temperature overnight, and solid precipitation was observed. *: no solid precipitation was observed in the sample after 2 days of slurrying; then the sample was transferred to temperature cycling and placed at -20 °C, and no solid was observed; then 0.5 mL of n-heptane (columns A and E)/MTBE (column B) was added, the mixture was slurried at room temperature overnight, no solid precipitation was observed; then the sample was transferred to room temperature for volatilization. | | | | | | |

### Example 6. Preparation of Crystal Form A of Tromethamine Salt of Compound of Formula I

The free acid crystal form A and an equimolar amount of tromethamine were slurried in MTBE at room temperature for 3 days, and the solid was separated out by centrifugation and dried under vacuum at room temperature to give crystal form A of a tromethamine salt of the compound of formula I.

The XRPD pattern of the crystal form A of the tromethamine salt sample is shown in FIG. 6. The TGA/DSC results are presented in FIGs. 7-8. The TGA result shows a weight loss of 2.41% when the sample was heated to 120 °C; the DSC result shows 1 endothermic peak at 139.2 °C (peak temperature) in the sample. ¹H NMR was measured in CD₃OD. The results are presented in FIG. 9. The results show that the molar ratio of tromethamine to free acid in the crystal form A of the tromethamine salt was 1:1, and no MTBE solvent residue was observed.

The XRPD analysis data of the crystal form A of the tromethamine salt of the compound of formula I obtained are shown in Table 6-1 below:

**Table 6-1. XRPD diffraction peak data of crystal form A of tromethamine salt**

| Peak number | 2θ [°] | Relative intensity [%] |
|---|---|---|
| 1 | 6.0250 | 37.14 |
| 2 | 9.0145 | 100.00 |
| 3 | 13.5026 | 2.69 |
| 4 | 15.0624 | 5.41 |
| 5 | 16.0258 | 1.18 |
| 6 | 18.0940 | 14.19 |
| 7 | 24.2684 | 23.15 |
| 8 | 27.2793 | 1.85 |
| 9 | 30.3859 | 1.27 |
| 10 | 36.7231 | 1.30 |

### Example 7. Preparation of Crystal Form A of Diethylamine Salt of Compound of Formula I

The free acid crystal form A sample and an equimolar amount of diethylamine were slurried in MTBE at room temperature for 3 days, and the solid was separated out by centrifugation and dried under vacuum at room temperature to give crystal form A of a diethylamine salt of the compound of formula I.

The XRPD pattern of the crystal form A of the diethylamine salt sample is shown in FIG. 10. The TGA/DSC results are detailed in FIGs. 11-12. The TGA result shows a weight loss of 1.80% when the sample was heated to 100.0 °C and a weight loss of 3.99% when the sample was heated from 100.0 °C to 210.0 °C; the DSC result shows 2 endothermic peaks at 104.3 °C and 121.5 °C (peak temperature) in the sample. ¹H NMR was measured in CD₃OD. The results are shown in FIG. 13. The results show that the molar ratio of diethylamine to free acid in the crystal form A of the diethylamine salt was 1:1, and no MTBE solvent residue was observed.

The XRPD analysis data of the crystal form A of the diethylamine salt of the compound of formula I obtained are shown in Table 7-1 below:

**Table 7-1. XRPD diffraction peak data of crystal form A of diethylamine salt**

| Peak number | 2θ [°] | Relative intensity [%] |
|---|---|---|
| 1 | 5.2862 | 100.00 |
| 2 | 9.7874 | 76.09 |
| 3 | 10.5332 | 10.81 |
| 4 | 11.2032 | 6.49 |
| 5 | 12.5208 | 3.55 |
| 6 | 14.8462 | 6.41 |
| 7 | 15.2041 | 11.29 |
| 8 | 16.2162 | 14.01 |
| 9 | 16.8594 | 9.00 |
| 10 | 18.3042 | 39.78 |
| 11 | 19.6099 | 14.00 |
| 12 | 19.9880 | 9.56 |
| 13 | 21.0950 | 29.76 |
| 14 | 22.1176 | 7.76 |
| 15 | 22.4689 | 20.23 |
| 16 | 24.5720 | 16.65 |
| 17 | 24.8493 | 8.87 |
| 18 | 25.3339 | 10.14 |
| 19 | 26.4478 | 12.13 |
| 20 | 27.3857 | 3.09 |
| 21 | 28.0090 | 3.04 |
| 22 | 29.6148 | 4.06 |
| 23 | 32.1328 | 1.74 |
| 24 | 34.9994 | 1.34 |
| 25 | 37.4445 | 0.95 |

### Example 8. Preparation of Crystal Form A of Lysine Salt of Compound of Formula I

The free acid crystal form A sample and an equimolar amount of lysine were slurried in EtOAc at room temperature for 3 days, and the solid was separated out by centrifugation and dried under vacuum at room temperature to give crystal form A of a lysine salt of the compound of formula I. The sample was characterized by XRPD (see FIG. 14).

The XRPD analysis data of the crystal form A of the lysine salt of the compound of formula I obtained are shown in Table 8-1 below:

**Table 8-1. XRPD diffraction peak data of crystal form A of lysine salt**

| Peak number | 2θ [°] | Relative intensity [%] |
|---|---|---|
| 1 | 5.1229 | 45.44 |
| 2 | 10.4385 | 100.00 |
| 3 | 15.5641 | 10.73 |
| 4 | 18.0728 | 60.12 |
| 5 | 19.6086 | 75.10 |
| 6 | 21.1013 | 57.18 |
| 7 | 24.2156 | 37.83 |
| 8 | 32.9708 | 12.35 |

### Example 9. Dynamic Solubility Experiment

The solid at a feeding concentration of 10 mg/mL (40 mg of solid in 4 mL of solvent, calculated as free acid) was rotationally mixed at 37 °C, and the solubility of each sample in four systems of water, SGF, FaSSIF, and FeSSIF was determined at different time points (1 h, 2 h, 4 h, and 24 h). After sampling at each time point, the sample was centrifuged (at 10000 rpm) and filtered (through a 0.45 µm PTFE filter head), the filtrate was determined for HPLC concentration and pH value, and the centrifuged solid samples were tested for XRPD. The solubility test results are summarized in Table 9-1, the solubility curves are shown in FIG. 15, and the XRPD results of the samples after the solubility tests are shown in FIGs. 16-21. The results show that the solubility of the crystal form A of the tromethamine salt in different vehicles was better than that of the free acid crystal form A, the crystal form of the free acid crystal form A was unchanged after the vehicle solubility tests, and the crystal form A of the tromethamine salt was converted into the free acid crystal form A after the SGF and FeSSIF solubility tests.

**Table 9-1. Dynamic solubility test results at 37 °C**

| **Starting material** | **Solvent** | **1 h** | | | **2 h** | | | **4 h** | | | **24 h** | | |
|---|---|---|---|---|---|---|---|---|---|---|---|---|---|
| | | S | pH | Crystal form change | S | pH | Crystal form change | S | pH | Crystal form change | S | pH | Crystal form change |
| Free acid crystal form A | H₂O | 0.08 | 8.3 | No | 0.08 | 8.4 | No | 0.09 | 8.3 | No | 0.13 | 4.8 | No |
| | SGF | 0.04 | 1.8 | No | 0.04 | 1.8 | No | 0.05 | 1.8 | No | 0.04 | 1.8 | No |
| | FaSSIF | 1.96 | 6.1 | No | 1.83 | 6.1 | No | 1.86 | 6.1 | No | 1.59 | 6.1 | No |
| | FeSSIF | 0.21 | 4.9 | No | 0.22 | 4.9 | No | 0.22 | 4.9 | No | 0.21 | 5.0 | No |
| Crystal form A of tromethamine salt | H₂O | 8.36 | 6.9 | -- | 8.36 | 6.9 | -- | 8.33 | 6.9 | -- | 7.82 | 6.8 | -- |
| | SGF | 2.35 | 6.3 | Yes*^{a}* | 2.85 | 6.4 | Yes^{a} | 2.85 | 6.3 | Yes*^{a}* | 2.93 | 6.3 | Yes^{a} |
| | FaSSIF | 8.88 | 6.5 | -- | 9.07 | 6.5 | -- | 9.15 | 6.5 | -- | 8.37 | 6.5 | -- |
| | FeSSIF | 2.04 | 5.3 | Yes*^{a}* | 2.19 | 5.3 | Yes^{a} | 2.17 | 5.3 | Yes^{a} | 0.52 | 5.4 | Yes^{a} |

| | | | | | | | | | | | | | |
|---|---|---|---|---|---|---|---|---|---|---|---|---|---|
| S: solubility (mg/mL); --: the remaining amount of the solid was relatively small, so XRPD was not tested; *^{a}*: it was converted to the free acid crystal form A. | | | | | | | | | | | | | |

Preparation description of biological vehicles:

### Preparation of simulated gastric fluid (SGF)

200.3 mg of NaCl and 105.0 mg of Triton X-100 were weighed out and added to a 50-mL volumetric flask, purified water was added, and the mixture was completely dissolved. 136 µL of concentrated hydrochloric acid (12 M) was added, and the pH was adjusted to 1.8 with 1 M hydrochloric acid or 1 M NaOH solution. Purified water was added, and the mixed solution was brought to volume.

### Preparation of fasted state simulated intestinal fluid (FaSSIF)

0.34 g of anhydrous NaH₂PO₄, 0.44 g of NaOH, and 0.62 g of NaCl were weighed out and added to a 50-mL volumetric flask. Purified water was added, the mixture was completely dissolved, and the pH was adjusted to 6.5 with 1 M hydrochloric acid or 1 M NaOH solution. Purified water was added, and the mixed solution was brought to volume. 0.11 g of SIF powder was then weighed out, and the mixed solution was completely dissolved.

### Preparation of fed state simulated intestinal fluid (FeSSIF)

0.82 mL of glacial acetic acid, 0.40 g of NaOH, and 1.19 g of NaCl were added to a 50-mL volumetric flask. About 48 mL of purified water was added, the mixture was completely dissolved, and the pH was adjusted to 5.0 with 1 M hydrochloric acid or 1 M NaOH solution. Purified water was added, and the mixed solution was brought to volume. 0.56 g of SIF powder was then weighed out, and the mixed solution was completely dissolved.

### Example 10. Hygroscopicity

Hygroscopicity evaluation was performed on the free acid crystal form A and the crystal form A of the tromethamine salt using a dynamic vapor sorption (DVS) instrument. Starting at 0% RH, the test acquired the percentage change in mass of the sample when humidity changed (0% RH to 95% RH to 0% RH) at a constant temperature of 25 °C. The DVS test results and XRPD results before and after DVS test are shown in FIGs. 22-25. The results show that the vapor sorption of the free acid crystal form A sample at 25 °C/80% RH was 0.07%, indicating that it has almost no hygroscopicity; the weight of the crystal form A of the tromethamine salt sample at 25 °C/80% RH was 0.6%, indicating that it has a slight hygroscopicity. The crystal forms of the free acid crystal form A and the crystal form A of the tromethamine salt were unchanged after DVS test.

### Example 11. Solid State Stability

The free acid crystal form A and the crystal form A of the tromethamine salt were left to stand uncovered for 1/4 weeks at 25 °C/60% RH and 40 °C/75% RH, respectively, and then the physical and chemical stability of the samples was tested by XRPD and HPLC. Purity data are shown in Table 11-1, and XRPD results are shown in FIGs. 26-27. The results show that the HPLC purities of the free acid crystal form A and the crystal form A of the tromethamine salt were not significantly changed after the placement under corresponding conditions, and the crystal forms were not changed.

**Table 11-1. Summary of solid state stability evaluation**

| **Crystal form (Batch No.)** | **Conditions** | | **HPLC results** | | |
|---|---|---|---|---|---|
| | | | Purity (area%) | Relative to starting (%) | Crystal form |
| Free acid crystal form A | Starting | | 94.35 | -- | -- |
| | 25 °C/60% RH/uncovered | 1 week | 94.17 | 99.81 | Free acid crystal form A |
| | | 4 weeks | 94.19 | 99.83 | |
| | 40 °C/75% RH/uncovered | 1 week | 94.15 | 99.79 | |
| | | 4 weeks | 94.13 | 99.77 | |
| Crystal form A of tromethamine salt | Starting | | 94.18 | -- | -- |
| | 25 °C/60% RH/uncovered | 1 week | 94.27 | 100.10 | Crystal form A of tromethamine salt |
| | | 4 weeks | 94.33 | 100.16 | |
| | 40 °C/75% RH/uncovered | 1 week | 94.26 | 100.08 | |
| | | 4 weeks | 94.35 | 100.18 | |

### Example 12. Different Preparation Processes for Free Acid Crystal Form A of Compound of Formula I

The compound of formula I was prepared according to the process method in Example 2, and the free acid crystal form A (identified by XRPD) was prepared according to the following methods.

12.1. To a 5 mL vial was added 20 mg of the compound of formula I, and 0.4-1.0 mL of a solvent was added. The mixture was dissolved and filtered, and the vial was sealed with a sealing film on which 2 holes were pricked, and left to stand at room temperature for slow volatilization. The resulting solid was collected. The solvent was selected from one of ethyl acetate, dichloromethane, methyl *tert-*butyl ether, and isopropanol/water (3:1).

12.2. To a 20 mL vial was added 20 mg of the compound of formula I, and 0.4-1.0 mL of a solvent was added. An anti-solvent (about 5 mL) was added to the clarified solution while stirring (1000 rpm) (an anti-solvent was added dropwise to the clarified solution while stirring (1000 rpm) until the solid was precipitated; or after the total volume of the anti-solvent was added to 5 mL, the sample without solid precipitation was suspended and stirred at 5 °C; if there was still no solid precipitated, the sample was suspended and stirred at -20 °C, and the final clarified sample volatilized at room temperature) until the solid was precipitated. The resulting solid was collected. When the dissolving solvent adopted methanol, the anti-solvent adopted water; when the dissolving solvent adopted methyl ethyl ketone, the anti-solvent adopted 4-cymene or *n*-heptane; when the dissolving solvent adopted isopropyl acetate, the anti-solvent adopted cyclohexane or *m*-xylene; when the dissolving solvent was selected from tetrahydrofuran, the anti-solvent adopted water or toluene; when the dissolving solvent was selected from methyl *tert*-butyl ether, the anti-solvent was selected from methylcyclohexane; when the dissolving solvent was selected from dimethylacetamide, the anti-solvent was selected from water.

12.3. 20 mg of the compound of formula I was completely dissolved in 0.4-0.6 mL of a solvent. The clarified solution was added dropwise to 5 mL of an anti-solvent while stirring (1000 rpm), and the precipitated solid was collected. When the dissolving solvent adopted one of isopropanol, ethyl acetate, or 2-methyltetrahydrofuran, the anti-solvent adopted n-heptane; when the dissolving solvent adopted one of acetone, *tert*-butyl acetate, or trichloromethane, the anti-solvent adopted toluene; when the dissolving solvent adopted acetonitrile, the anti-solvent adopted water.

12.4. About 20-90 mg of the compound of formula I was weighed out and added to an HPLC glass vial, and 0.5 mL of a solvent was added. The resulting turbid solution was placed at room temperature under magnetic stirring (1000 rpm) for about 4 days, and then centrifuged (10000 rpm, 2 min) to collect the solid. The solvent was selected from one of *n*-pentane, *m*-xylene, isopropyl ether, cyclohexane, water, methanol/water (1:5), *N,N*-dimethylformamide/water (1:4), 2-methyltetrahydrofuran/*n*-hexane (1:5), acetone/*n*-heptane (1:4), anisole/*n*-heptane (1:4), methyl acetate/cyclohexane (1:4), methanol/4-cymene (1:4), dichloromethane/methyl *n*-hexane (1:5), and acetonitrile/toluene (1:4).

12.5. About 20-50 mg of the compound of formula I was weighed out and added to an HPLC glass vial, and 0.5 mL of a solvent was added. The resulting suspension was magnetically stirred (1000 rpm) at 50 °C for about 3 days, and then centrifuged (10000 rpm, 2 min) to collect the solid. The solvent was selected from one of methylcyclohexane, cumene, water, 1,4-dioxane/water (1:9), dimethylacetamide/water (1:9), tetrahydrofuran/*n*-hexane (1:9), 2-methyltetrahydrofuran/cyclohexane (1:4), cyclopentyl methyl ether/*n*-pentane (1:4), methyl ethyl ketone/cumene (1:7), isopropyl acetate/toluene (1:9), isobutanol/4-cymene (1:4), and trichloromethane/*m*-xylene (1:4).

12.6. About 20 mg of the compound of formula I was weighed out and added to an HPLC glass vial, and 0.5 mL of a solvent was added. The resulting suspension was magnetically stirred (1000 rpm) at temperature cycles (50 °C-5 °C, 0.1 °C/min, 2 cycles) and then centrifuged (10000 rpm, 2 min) to collect the solid. The solvent was selected from one of toluene, *n*-heptane, 4-cymene, ethanol/water (1:4), ethyl acetate/methylcyclohexane (1:9), cyclopentyl methyl ether/*n*-hexane (1:3), trichloromethane/*n*-heptane (1:7), and methyl ethyl ketone/*m*-xylene (1:9).

12.7. About 20 mg of the compound of formula I was weighed out and added to a mortar, and 0.2 mL of a solvent was added. The sample was ground for 2-3 min, and the solid was collected. The solvent was selected from one of ethanol, dichloromethane, ethyl acetate, and tetrahydrofuran.

The above examples illustrate the embodiments of the present disclosure. However, the present disclosure is not limited to the embodiments described above. Any modification, equivalent replacement, improvement, and the like made without departing from the spirit and principle of the present disclosure shall fall within the protection scope of the present disclosure.

## Claims

1. A crystal form of a compound of formula I, wherein the compound of formula I has a structure as follows:

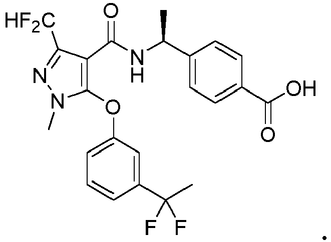

2. The crystal form of the compound of formula I according to claim 1, wherein the crystal form is free acid crystal form A of the compound of formula I, and the free acid crystal form A has an X-ray powder diffraction pattern comprising diffraction peaks at 2θ±0.2° diffraction angles of 7.88, 11.00, 12.13, 16.10, 19.75, 20.65, 21.04, 22.92, 23.53, and 26.69; or the free acid crystal form A has an X-ray powder diffraction pattern comprising diffraction peaks at 2θ±0.2° diffraction angles of 7.88, 8.08, 11.00, 12.13, 16.10, 19.75, 20.65, and 23.53; further, the free acid crystal form A has an X-ray powder diffraction pattern comprising diffraction peaks at 2θ±0.2° diffraction angles of 7.88, 8.08, 11.00, 12.13, 13.59, 15.50, 16.10, 19.44, 19.75, 20.65, 21.04, 22.92, 23.53, 25.32, 26.44, 26.69, and 27.67; furthermore, the free acid crystal form A has an X-ray powder diffraction pattern comprising diffraction peaks at 2θ±0.2° diffraction angles of 7.88, 8.08, 10.31, 10.49, 11.00, 11.46, 12.13, 12.82, 13.59, 15.29, 15.50, 15.72, 16.10, 18.27, 18.70, 19.04, 19.44, 19.75, 20.65, 21.04, 21.62, 21.97, 22.27, 22.92, 23.53, 24.22, 25.32, 25.81, 26.21, 26.44, 26.69, 27.67, 28.99, 29.35, 30.17, 31.12, 31.65, 32.18, 33.44, 33.97, 35.78, 36.84, 37.18, and 37.99; still further, the free acid crystal form A has an XRPD pattern substantially shown in FIG. 1;
preferably, the free acid crystal form A has one, two, or three of the following characteristics:
(1) a TGA curve of the free acid crystal form A showing a weight loss of about 0-2%, preferably about 0-1% (e.g., 0.84%) at 150.0±3 °C;
(2) a DSC curve of the free acid crystal form A having a starting point of an endothermic peak at 134.0±3 °C; and
(3) the DSC curve of the free acid crystal form A having an endothermic peak at 136.8±3 °C.

3. The crystal form of the compound of formula I according to claim 1, wherein the crystal form is free acid crystal form B of the compound of formula I, and the free acid crystal form B has an X-ray powder diffraction pattern comprising diffraction peaks at 2θ±0.2° diffraction angles of 7.38, 8.91, 11.07, 17.85, 18.52, 19.38, 23.05, 26.01, and 26.76; further, the free acid crystal form B has an X-ray powder diffraction pattern comprising diffraction peaks at 2θ±0.2° diffraction angles of 7.38, 8.91, 11.07, 12.01, 17.85, 18.52, 19.38, 23.05, 26.01, and 26.76; further, the free acid crystal form B has an X-ray powder diffraction pattern comprising diffraction peaks at 2θ±0.2° diffraction angles of 5.56, 6.00, 7.38, 8.91, 11.07, 11.58, 12.01, 13.58, 14.16, 14.78, 17.85, 18.52, 19.38, 22.33, 23.05, 24.64, 26.01, and 26.76; furthermore, the free acid crystal form B has an X-ray powder diffraction pattern comprising diffraction peaks at 2θ±0.2° diffraction angles of 5.56, 6.00, 7.38, 8.27, 8.91, 11.07, 11.58, 12.01, 13.58, 14.16, 14.78, 15.73, 17.85, 18.52, 19.38, 22.33, 23.05, 24.64, 26.01, and 26.76; still further, the free acid crystal form B has an XRPD pattern substantially shown in FIG. 5.

4. A pharmaceutically acceptable salt of a compound of formula I, or a crystal form thereof, wherein the pharmaceutically acceptable salt is selected from an alkali metal salt (e.g., a sodium salt or potassium salt), an alkaline earth metal salt (e.g., a calcium salt or magnesium salt), or an ammonium salt of the compound of formula I, or may be selected from a salt formed with an organic base that provides a physiologically acceptable cation, such as a salt formed with the following bases: sodium hydroxide, potassium hydroxide, calcium hydroxide, magnesium hydroxide, zinc hydroxide, betaine, monoethanolamine, caffeine, urea, nicotinamide, isonicotine, dimethylglucamine, ethylglucamine, glucosamine, meglumine, lysine, arginine, choline, aqueous ammonia, dicyclohexylamine, 1,6-hexanediamine, ethanolamine, diethanolamine, sarcosine, serinol, tris(hydroxymethyl)aminomethane, aminopropanediol, tromethamine, diethylamine, and imidazole; preferably, the pharmaceutically acceptable salt of the compound of formula I is a tromethamine salt, a diethylamine salt, and a lysine salt of the compound of formula I; the compound of formula I has a structure as follows:

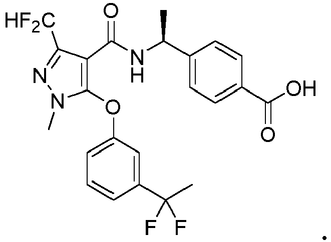

5. The crystal form of the pharmaceutically acceptable salt of the compound of formula I according to claim 4, wherein the crystal form is crystal form A of the tromethamine salt of the compound of formula I, and the crystal form A of the tromethamine salt has an X-ray powder diffraction pattern comprising diffraction peaks at 2θ±0.2° diffraction angles of 6.03, 9.01, 13.50, 15.06, 18.09, and 24.27; further, the crystal form A of the tromethamine salt has an X-ray powder diffraction pattern comprising diffraction peaks at 2θ±0.2° diffraction angles of 6.03, 9.01, 13.50, 15.06, 16.03, 18.09, 24.27, 27.28, 30.39, and 36.72; furthermore, the crystal form A of the tromethamine salt has an XRPD pattern substantially shown in FIG. 6; preferably, the crystal form A of the tromethamine salt has one, two, or three of the following characteristics:
(1) a TGA curve of the crystal form A of the tromethamine salt showing a weight loss of about 1.0%-3.5%, preferably about 2.0%-3.0% (e.g., 2.41%) at 120.0±3 °C;
(2) a DSC curve of the crystal form A of the tromethamine salt having a starting point of an endothermic peak at 124.5±3 °C; and
(3) the DSC curve of the free acid crystal form A having an endothermic peak at 139.2±3 °C; more preferably, the TGA profile of the crystal form A of the tromethamine salt is shown in FIG. 7; the DSC profile of the crystal form A of the tromethamine salt is shown in FIG. 8; a ¹H NMR spectrum of the crystal form A of the tromethamine salt is shown in FIG. 9.

6. The crystal form of the pharmaceutically acceptable salt of the compound of formula I according to claim 4, wherein the crystal form is crystal form A of the diethylamine salt of the compound of formula I, and the crystal form A of the diethylamine salt has an X-ray powder diffraction pattern comprising diffraction peaks at 2θ±0.2° diffraction angles of 5.29, 9.79, 10.53, 18.30, 19.61, 19.99, 21.10, 25.33, and 26.45; further, the crystal form A of the diethylamine salt has an X-ray powder diffraction pattern comprising diffraction peaks at 2θ±0.2° diffraction angles of 5.29, 9.79, 10.53, 11.20, 12.52, 14.85, 15.20, 16.22, 16.86, 18.30, 19.61, 19.99, 21.10, 22.12, 22.47, 24.57, 24.85, 25.33, 26.45, 27.39, 28.01, 29.61, 32.13, 35.00, and 37.44; furthermore, the crystal form A of the diethylamine salt has an XRPD pattern substantially shown in FIG. 10;
preferably, the crystal form A of the diethylamine salt has one or two of the following characteristics:
(1) a TGA curve of the crystal form A of the diethylamine salt showing a weight loss of about 0.50%-3.00%, preferably about 1.00%-2.50% (e.g., 1.80%) at 100.0±3 °C; and showing a weight loss of about 2.00%-5.00%, preferably about 3.00%-4.50% (e.g., 3.99%) at 210.0±3 °C; and
(2) a DSC curve of the crystal form A of the diethylamine salt having two endothermic peaks at 104.3±10 °C and 121.5±10 °C; particularly, the DSC curve of the crystal form A of the diethylamine salt having two endothermic peaks at 104.3±5 °C and 121.5±5 °C;
more preferably, the TGA profile of the crystal form A of the diethylamine salt is shown in FIG. 11; the DSC profile of the crystal form A of the diethylamine salt is shown in FIG. 12; a ¹H NMR spectrum of the crystal form A of the diethylamine salt is shown in FIG. 13.

7. The crystal form of the pharmaceutically acceptable salt of the compound of formula I according to claim 4, wherein the crystal form is crystal form A of the lysine salt of the compound of formula I, and the crystal form A of the lysine salt has an X-ray powder diffraction pattern comprising diffraction peaks at 2θ±0.2° diffraction angles of 5.12, 10.44, 15.56, 18.07, 19.61, and 21.10; further, the crystal form A of the lysine salt has an X-ray powder diffraction pattern comprising diffraction peaks at 2θ±0.2° diffraction angles of 5.12, 10.44, 15.56, 18.07, 19.61, 21.10, 24.22, and 32.97; furthermore, the crystal form A of the lysine salt has an XRPD pattern substantially shown in FIG. 14.

8. A preparation method for the free acid crystal form A of the compound of formula I according to claim 2, comprising the following methods,
wherein Method 1 comprises: adding the compound of formula I into an organic solvent I, and volatilizing at room temperature, wherein the organic solvent I may be selected from one or a mixture of more of ethyl acetate, dichloromethane, methyl tert-butyl ether, isopropanol, and water;
Method 2 comprises: performing anti-solvent addition crystallization on the compound of formula I in an organic solvent II; if no solid is precipitated by stirring, adding an anti-solvent into the system, wherein the organic solvent II may be selected from one or a mixture of more of methanol, methyl ethyl ketone, isopropyl acetate, tetrahydrofuran, methyl *tert*-butyl ether, and dimethylacetamide; the anti-solvent may be selected from one or a mixture of more of water, isopropyl ether, toluene, *m-*xylene, 4-cymene, *n*-pentane, *n*-heptane, cyclohexane, and methylcyclohexane;
Method 3 comprises: suspending and stirring the compound of formula I in an organic solvent III at room temperature for crystallization, wherein the organic solvent III may be selected from one or a mixture of more of *n*-pentane, toluene, *m*-xylene, isopropyl ether, *n*-hexane, cyclohexane, methylcyclohexane, water, methanol, *N,N*-dimethylformamide, 2-methyltetrahydrofuran, acetone, methyl acetate, dichloromethane, and acetonitrile;
Method 4 comprises: suspending and stirring the compound of formula I in an organic solvent IV at 40-60 °C (e.g., 50 °C) for crystallization, wherein the organic solvent IV may be selected from one or a mixture of more of methylcyclohexane, cumene, water, 1,4-dioxane, dimethylacetamide, tetrahydrofuran, *n*-hexane, 2-methyltetrahydrofuran, *n*-pentane, methyl ethyl ketone, isopropyl acetate, toluene, isobutanol, trichloromethane, and *m*-xylene; and
Method 5 comprises: performing wet grinding on the compound of formula I in an organic solvent V for crystallization, wherein the organic solvent V may be selected from one or a mixture of more of ethanol, dichloromethane, ethyl acetate, and tetrahydrofuran.

9. A preparation method for the free acid crystal form B of the compound of formula I according to claim 3, comprising the following steps: dissolving the free acid crystal form A of the compound of formula I in an organic solvent B1, and then performing gas-liquid diffusion in an atmosphere of an organic solvent B2, wherein the organic solvent B1 is selected from ketones, such as methyl ethyl ketone, methyl isopropyl ketone, acetone, diethyl ketone, dipropyl ketone, diisopropyl ketone, dibutyl ketone, and diisobutyl ketone; the organic solvent B2 is selected from alkane organic compounds, preferably C1-C7 alkane organic compounds, for example, it is selected from n-pentane, *n*-heptane, and cyclohexane; preferably, the free acid crystal form A of the compound of formula I is dissolved in methyl isobutyl ketone (MIBK), and then the gas-liquid diffusion is performed in an *n*-pentane atmosphere to give the free acid crystal form B of the compound of formula I.

10. A preparation method for the pharmaceutically acceptable salt or the crystal form thereof of the compound of formula I according to any one of claims 4-7, comprising the following step: mixing the compound of formula I with a salt-forming reagent (such as a corresponding base) in a suitable solvent, wherein preferably, the solvent is selected from one or a mixture of more of ethanol, heptane, ethyl acetate, MTBE, acetonitrile, water, and acetone.

11. A pharmaceutical composition comprising one or more of the crystal form of the compound of formula I, or the pharmaceutically acceptable salt or the crystal form thereof of the compound of formula I according to any one of claims 1-7.

12. Use of the crystal form of the compound of formula I, or the pharmaceutically acceptable salt or the crystal form thereof of the compound of formula I according to any one of claims 1-7, or the pharmaceutical composition according to claim 11 for the manufacturing of a medicament for treating or preventing a disease associated with EP4.

13. The use according to claim 12, wherein the disease associated with EP4 comprises at least one selected from the following: inflammatory diseases, pain, cancers, metabolic diseases, and urinary system diseases; the inflammatory disease includes at least one selected from the following: arthritis and rheumatoid arthritis; the pain includes osteoarthritis pain and pain caused by endometriosis.

14. The use according to claim 12 or 13, wherein the crystal form of the compound of formula I, the pharmaceutically acceptable salt or the crystal form thereof of the compound of formula I, or the pharmaceutical composition may be administered in combination with radiotherapy and/or antibody therapy, wherein the antibody therapy is selected from one or a combination of CTLA4 antibody therapy, PDL1 antibody therapy, and PD1 antibody therapy.

15. A method for treating or preventing a disease associated with EP4 comprising administering to a patient a therapeutically effective dose of the free acid crystal form (e.g., the free acid crystal form A or the free acid crystal form B) of the compound of formula I, or the pharmaceutically acceptable salt (including the crystal form thereof) of the compound of formula I according to any one of claims 1-7, or the pharmaceutical composition according to claim 11.

16. The method according to claim 15, wherein the disease associated with EP4 comprises at least one selected from the following: inflammatory diseases, pain, cancers, metabolic diseases, and urinary system diseases; the inflammatory disease includes at least one selected from the following: arthritis and rheumatoid arthritis; the pain includes osteoarthritis pain and pain caused by endometriosis.
